# EUROPEAN PATENT SPECIFICATION

(11) **EP 1 308 174 B1**
(45) Date of publication and mention of the grant of the patent: **10.01.2007**
(21) Application number: 02257583.1
(22) Date of filing: 01.11.2002
(51) Int. Cl.: A61L 2/28, A61L 2/20, A61L 2/24, G01N 25/28, G01N 25/32, G01N 31/22

(54) **Method and apparatus for monitoring sterilant concentration in diffusion-restricted regions**
Verfahren und Vorrichtung zur Überwachung der Konzentration des Sterilisierungsmittels in diffusionbeschränkten Bereichen
Méthode et dispositif de surveillance de la concentration d'un agent de stérilisation dans des régions à diffusion limitée

(30) Priority: 02.11.2001 US 16058
(43) Date of publication of application: 07.05.2003
(73) Proprietor: ETHICON, Somerville, NJ 08876 (US)
(72) Inventor: Hui, Henry K., Laguna Niguel, CA 92677 (US); Lin, Szu-Min, Laguna Hills, CA 92653 (US); Timm, Debra, Foothill Ranch, CA 92610 (US); Lemus, Anthony, Villa Park, CA 92861 (US); Fryer, Ben, Lake Forest, CA 92630 (US); Engstrom, Keith, Laguna Niguel, CA 92677 (US)
(74) Representative: Fisher, Adrian John

(56) References cited:
- WO-A-01/45754
- GB-A- 2 191 585

## Description

### Background of the Invention

### Field of the Invention

The present invention relates generally to methods of sterilizing articles using an oxidative gas or vapor, and more particularly, to methods of monitoring the concentration of the oxidative gas or vapor during the sterilization process.

### Description of the Related Art

Chemical sterilization has been successfully used for the sterilization of medical devices to minimize damage to the medical devices during sterilization. Chemical sterilization uses a sterilizing fluid such as hydrogen peroxide, ethylene oxide, chlorine dioxide, formaldehyde, or peracetic acid in a sealed chamber to sterilize medical instruments. One commercial form of chemical sterilization is the STERRAD® Sterilization System, available through Advanced Sterilization Products of Irvine, California, a division of Ethicon, Inc. The STERRAD® Process utilizes hydrogen peroxide and low temperature gas plasma to sterilize medical devices.

The STERRAD® Sterilization Process is performed in the following manner. The load to be sterilized is placed in a sterilization chamber, the chamber is closed, and a vacuum is drawn. An aqueous solution of hydrogen peroxide is injected and vaporized into the chamber. A low-temperature gas plasma is initiated by applying an electric field to create a plasma. The hydrogen peroxide vapor dissociates in the plasma into reactive species that react with and kill microorganisms. After the activated components react with the organisms, surfaces in the chamber, or with each other, they lose their high energy and recombine to form oxygen, water, and other nontoxic byproducts. At the completion of the process, the plasma is turned off, the vacuum is released, and the chamber is returned to atmospheric pressure by venting.

In order for the sterilization process to be effective, the load to be sterilized must be exposed to a sufficient concentration of hydrogen peroxide. If the equipment in the chamber reacts with, absorbs, adsorbs, or condenses the hydrogen peroxide, there may not be sufficient hydrogen peroxide remaining for the sterilization process to be effective. The concentration of hydrogen peroxide in the chamber is therefore monitored to assure that sufficient hydrogen peroxide is present. If too much hydrogen peroxide is removed from the chamber through absorption, adsorption, condensation, or reaction with the equipment in the chamber, the cycle is cancelled, the remaining hydrogen peroxide in the chamber is removed by evacuating the chamber and/or introducing plasma to decompose the hydrogen peroxide, and a new cycle is started.

For example, Cummings, et al. (U.S. Patent No. 4,956,145) describe a method in which the hydrogen peroxide concentration is monitored, and additional hydrogen peroxide is added to maintain the concentration of hydrogen peroxide at a level which is effective for sterilization but is less than the saturation limit. Cummings, et al. did not describe any method for determining whether the equipment in the sterilization chamber significantly absorbs, adsorbs, condenses, or decomposes large amounts of hydrogen peroxide, however. If hydrogen peroxide is absorbed, adsorbed, or condensed onto the equipment, it may take a great deal of time to remove the hydrogen peroxide so that the equipment may be safely removed from the chamber.

Biological indicators have been used previously to monitor the efficacy of sterilization systems. Biological indicators typically include a microorganism source with a predetermined concentration of live microorganisms dried onto a substrate. The microorganism-impregnated substrate is placed in the loaded sterilization system and is subjected to a full sterilization process. Thereafter, the substrate is placed in a sterile culture medium and incubated for a predetermined time at an appropriate temperature with an indicator to indicate the presence or absence of viable microorganisms. At the end of the incubation period, the culture medium is examined to determine whether any microorganisms survived the sterilization process. Microorganism survival means that the sterilization was incomplete. Self-contained biological indicators have the microorganism source, culture medium, and indicator packaged together in a way that permits the microorganism source, culture, and indicator to be combined without exposing the biological indicator to non-sterile surroundings. Examples of such biological indicators are disclosed by Falkowski, et al. (U.S. Patent No. 5,801,010) and Smith (U.S. Patent No. 5,552,320).

In practice, biological indicators are placed in regions of the load which are anticipated to be especially resistant to the sterilization process. For example, certain loads include diffusion-restricted regions to be sterilized which are reached by the hydrogen peroxide only after diffusing through small openings or along long, narrow diffusion paths, such as lumens. Biological indicators can be made small enough to fit into most of these diffusion-restricted regions or environments. If the microorganisms of a biological indicator placed in such a region are killed by the sterilization process, then the sterilization process is deemed to be performing correctly. Using this method to determine whether the sterilization process was successful, however, produces an answer only after the incubation period.

WO 01/45754 discloses a method of the type set out in the preamble of the accompanying claim 1, and an apparatus of the type set out in the preamble of the accompanying claim 21.

### Summary of the Invention

In one aspect, the present invention provides a method of monitoring a concentration of an oxidative gas or vapor in a diffusion-restricted region in fluid communication with a sterilization chamber during a sterilization process as set forth in the accompanying claim 1.

In another aspect, the present invention provides an apparatus for monitoring a concentration of an oxidative gas or vapor in a sterilization chamber during a sterilization process as set forth in the accompanying claim 21.

### Brief Description of the Drawings

Figures 1A 1B, 1C, 1D, and 1E schematically illustrate various known concentration monitors which comprise a carrier, a chemical substance, and a temperature probe.

Figure 2 schematically illustrates a known sterilization system.

Figures 3A, 3B, 3C, 3D, and 3E schematically illustrate various concentration monitors comprising a reference temperature probe. The concentration monitor of Figure 3A is known. The concentration monitors of Figures 3B-3E comprise alternative embodiments of the present invention.

Figure 4A schematically illustrates a concentration monitor comprising an integrated circuit chip.

Figure 4B schematically illustrates a concentration monitor comprising thermocouple junctions comprising thin conductive films.

Figure 5 schematically illustrates a sterilization system as disclosed in the prior art.

Figure 6 schematically illustrates a test pack as disclosed in the prior art.

Figure 7 is a flow diagram of a known method of determining a concentration of an oxidative gas or vapor in a diffusion-restricted region.

Figure 8 schematically illustrates a diffusion-restricted region and concentration monitor compatible with embodiments of the present invention.

Figure 9 schematically illustrates a test pack placed in a sterilization system with the load in accordance with embodiments of the present invention.

Figure 10 is a flow diagram of a method of determining the suitability of a load for sterilization with an oxidative gas or vapor in accordance with another embodiment of the present invention.

Figure 11 schematically illustrates a portion of a concentration monitor inside a lumen in accordance with embodiments of the present invention.

Figure 12 schematically illustrates a portion of a concentration monitor inside a lumen placed inside a container comprising openings covered by a gas-permeable material in accordance with embodiments of the present invention.

Figure 13 schematically illustrates a portion of a concentration monitor inside a process challenge device (PCD) in accordance with embodiments of the present invention.

Figure 14 schematically illustrates a portion of a concentration monitor inside a second chamber in fluid communication with the sterilization chamber via a conduit.

Figure 15 schematically illustrates a portion of a concentration monitor inside a package comprising a gas-permeable portion and containing a device in accordance with embodiments of the present invention.

### Detailed Description of the Preferred Embodiment

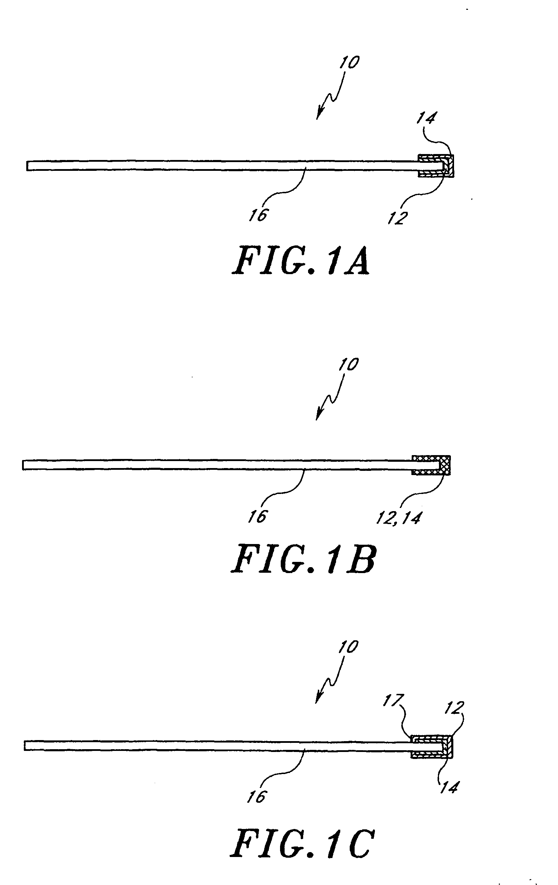

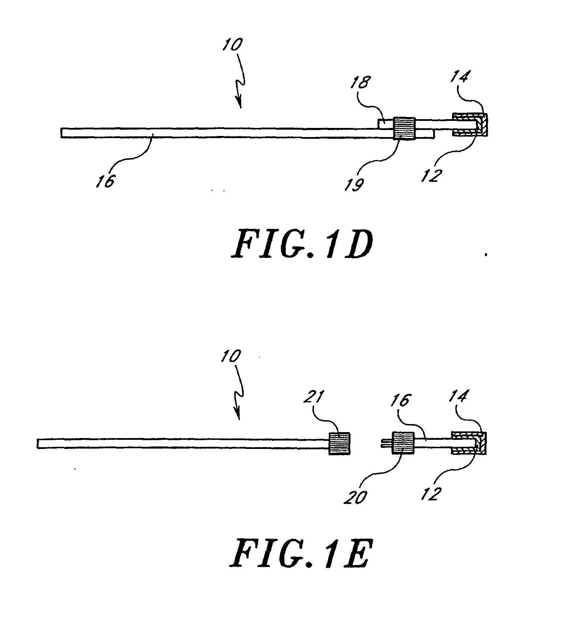

Figures 1A, 1B, 1C, 1D, and 1E each illustrate a known concentration monitor 10 compatible with embodiments of the present invention. The concentration monitor 10 comprises a carrier 12, a chemical substance 14, and a temperature probe 16. All of the elements of the concentration monitor 10 must be compatible with its operating conditions. Concentration monitors 10 can operate under a wide range of pressures, such as atmospheric pressures or subatmospheric pressures (i.e., vacuum pressures). For use in a sterilization system utilizing hydrogen peroxide vapor with or without plasma, the carrier 12, chemical substance 14, and temperature probe 16 must all be compatible with operations under sterilization conditions and with exposure to hydrogen peroxide vapor and plasma. Persons skilled in the art recognize that there is a wide variety of materials and structures which can be selected as the carrier 12 in these embodiments. The carrier 12 couples the chemical substance 14 in close proximity to the temperature probe 16 so as to minimize the thermal losses between them. Examples of adequate carriers include, but are not limited to, acrylic, epoxy, nylons, polyurethane, polyhydroxyethylenemethacrylate (polyHEMA), polymethylmethacrylate (PMMA), polyvinylpyrrolidone (PVP), polyvinylalcohol (PVA), silicone, tape, or vacuum grease. Additionally, the carrier 12 can either be configured to expose the chemical substance 14 directly to the environment, or to enclose the chemical substance 14 in a gas permeable pouch, such as Tyvek (RTM) tubing, or a gas impermeable enclosure with a hole or holes. In certain embodiments, the chemical substance 14 can be coupled directly to the temperature probe 16 without use of a carrier. For example, the chemical substance 14 can be formed as an integral part of the temperature probe 16 or, if the chemical substance 14 is sufficiently adhesive, it can be directly coupled to the temperature probe 16. Chemical vapor deposition or electrochemical plating can also be used to couple the chemical substance 14 directly to the temperature probe 16.

The chemical substance 14 undergoes an exothermic reaction with the oxidative gas or vapor to be monitored, producing a detectable amount of thermal energy (i.e., heat) upon exposure to the oxidative gas or vapor to be monitored. Persons skilled in the art are able to choose an appropriate chemical substance 14 which yields a sufficient amount of heat upon exposure to the relevant range of concentrations of the oxidative gas or vapor to be measured. Examples of chemical substances 14 for use in a hydrogen peroxide sterilization system include, but are not limited to, substances that catalytically decompose hydrogen peroxide, substances that are easily oxidized by hydrogen peroxide, and substances that contain hydroxyl functional groups. Substances that catalytically decompose hydrogen peroxide include, but are not limited to, catalase, copper and copper alloys, iron, silver, platinum, and palladium. Substances that are easily oxidized by hydrogen peroxide include, but are not limited to, magnesium chloride (MgCl₂), iron (II) compounds such as iron (II) acetate, potassium iodide (KI), sodium thiosulfate, and sulfides and disulfides such as molybdenum disulfide, 1,2-ethanedithiol, methyl disulfide, cysteine, methionine, and polysulfides. Substances that contain hydroxyl functional groups include, but are not limited to, polyethylene glycol (PEG), polyethylene oxide (PEO), and polyvinyl alcohol (PVA). These substances can be in the form of polymers that comprise hydroxyl functional groups, and persons skilled in the art appreciate that such polymers can also be co-polymers. In addition, a combination of these above-described substances may be chosen as the chemical substance 14. Furthermore, persons skilled in the art are able to select the appropriate amount of chemical substance 14 to yield a sufficient amount of heat upon exposure to the relevant range of hydrogen peroxide concentrations.

Various known configurations compatible with use with embodiments of the present invention are illustrated in Figures 1A, 1B, 1C, 1D, and 1E. Figure 1A shows a temperature probe 16 coated with a thin layer of carrier 12 on the tip of the probe 16 and the chemical substance 14 is coated on the outside of the carrier 12. Figure 1B shows the chemical substance 14 is mixed with the carrier 12 and applied onto the tip of the temperature probe 16. For example, a chemical substance 14 such as PEG is mixed with a carrier 12 such as acrylic binder in an aqueous suspension, then coated onto a temperature probe 16. The chemical substance 14 is accessible for reaction as the hydrogen peroxide diffuses into the carrier. Figure 1C show the chemical substance 14 is enclosed onto the tip of the temperature probe 16 with a carrier 12. The carrier 12 is a gas-permeable pouch with a heat-sealed area 17, which typically is composed of a nonwoven polyolefin material, such as Tyvek® (nonwoven polyethelene) sold by E.I. du Pont de Nemours and Co. of Wilmington, Delaware or CSR (central supply room) wrapping material (nonwoven polypropylene) sold by Kimberly-Clark Corp. of Dallas, Texas. The carrier 12 can also be a gas-impermeable pouch or other enclosure with one or more holes to allow the diffusion of gas or vapor to react with the chemical substance 14 retained in the enclosure. Figure 1D shows a chemical substance 14 coupled to a heat-conducting material 18 with a carrier 12, and the heat-conducting material 18 is coupled to the temperature probe 16 with a substrate 19. The substrate 19 can be tape, adhesive, or any other coupling means. The heat-conducting material 18 can be metallic wire or any other materials which can properly conduct heat to the temperature probe 16. Figure 1E shows a chemical substance 14 coupled to a temperature probe 16 with a carrier 12, and two parts of the temperature probe 16 can be connected and disconnected with a male connector 20 and a female connector 21.

The temperature probe 16 is a device which measures the temperature at a particular location. One embodiment of the present invention utilizes a fiberoptic temperature probe, such as a Luxtron® 3100 fluoroptic thermometer, as the temperature probe 16. This fiberoptic temperature probe 16 is coated with Teflon (RTM) and therefore is very compatible to any oxidative gas or vapor. Another embodiment utilizes a temperature probe 16 which is a thermocouple probe which utilizes a junction of two metals or alloys. The thermocouple junction produces a voltage which is a known function of the junction's temperature. Measurements of this voltage across the thermocouple junction can therefore be converted into measurements of the junction's temperature. Thermocouple junctions can be made quite small (e.g., by spot welding together two wires of 0.025-millimeter diameter composed of differing alloys), so they can be positioned into size-restricted volumes. In yet other embodiments, the temperature probe 16 can be a thermistor, glass thermometer, resistance temperature detector (RTD) probe, temperature strip, optical temperature sensor, or infrared temperature sensor.

Table 1 illustrates the increases of temperature measured by a concentration monitor 10 with potassium iodide (KI) as the chemical substance 14. The tip of the fiberoptic temperature probe was first coated with a thin layer of Dow Corning high vacuum grease (part number 2021846-0888). About 0.15 grams of KI powder was then applied onto the vacuum grease. This configuration is the same as illustrated in Figure 1A. The measurements were conducted by suspending the concentration monitor 10 in a vacuum chamber heated to 45 °C, evacuating the chamber, recording the initial probe temperature, injecting hydrogen peroxide into the chamber, recording the temperature after all hydrogen peroxide was vaporized, evacuating the chamber to remove the hydrogen peroxide, and venting the chamber. The measurements were repeated with different concentrations of hydrogen peroxide injected into the chamber. The same temperature probe 16 was reused for all the measurements, and the results are shown in Table 1. As can be seen from Table 1, KI produces a measurable increase of temperature with increasing concentration of hydrogen peroxide. Additionally, this concentration monitor 10 can be reused many times.

**Table 1:**

| Concentration of H₂O₂ (mg/L) | Temperature increase (°C) |
|---|---|
| 0.2 | 3.0 |
| 0.4 | 8.3 |
| 0.8 | 19.2 |
| 1.3 | 24.2 |
| 2.1 | 33.7 |

Table 2 provides data on the measured temperature increases with varying concentrations of hydrogen peroxide for a concentration monitor 10 utilizing different chemical substances 14. Same test conditions and probe configurations were used in these temperature measurements. As can be seen from Table 2, each of the chemical substances 14 produced a measurable temperature rise which increased with increasing hydrogen peroxide concentration.

**Table 2:**

| Chemical substance | Temperature increase (°C) | | |
|---|---|---|---|
| | 0.4 mg/L | 1.0 mg/L | 2.1 mg/L |
| Platinum on Alumina | 13.5 | 17.2 | --- |
| Catalase | 1.1 | --- | 6.9 |
| Iron (II) acetate | 62.5 | 83.1 | --- |
| Magnesium Chloride | 0.8 | --- | 4.4 |

The utility of using a thermocouple junction as the temperature probe 16 is illustrated in Table 3. For these measurements, the concentration monitor 10 was configured as illustrated in Figure 1A. The test conditions of Table 1 were also used for these measurements. Table 3 illustrates that significant temperature increases were also observed using a thermocouple temperature probe 16.

**Table 3:**

| Concentration of H₂O₂ (mg/L) | Temperature increase (°C) |
|---|---|
| 0.2 | 2.7 |
| 0.4 | 11.9 |
| 0.8 | 19.3 |
| 2.1 | 24.2 |

The utility of using double-sided tape as the carrier 12 is illustrated by Table 4, which presents the temperature increases measured by a fiberoptic temperature probe 16. A thin layer of 3M Scotch (RTM) double-sided tape was first applied to the tip of the fiberoptic probe 16. About 0.15 grams of KI powder was then coated onto the tape. Table 1 test conditions were repeated for these measurements. It is apparent from Table 4 that measurable increases of temperature were detected for increasing H₂O₂ concentration when using double-sided tape as the carrier 12.

**Table 4:**

| Concentration of H₂O₂ (mg/L) | Temperature increase (°C) |
|---|---|
| 0.4 | 9.3 |
| 1 | 16.8 |
| 2.1 | 31.2 |

The utility of using epoxy as the carrier 12 is illustrated by Table 5, which presents the temperature increases measured by a fiberoptic temperature probe 16. The concentration monitor 10 was constructed by applying a thin layer of Cole-Palmer 8778 epoxy on an aluminum wire. About 0.15 grams of KI powder was then applied and dried onto the epoxy. Finally, the aluminum wire was attached to the temperature probe 16. Table 1 test conditions were repeated for these measurements. It is apparent that measurable increases of temperature were detected for increasing H₂O₂ concentration when using epoxy as the carrier 12.

**Table 5:**

| Concentration of H₂O₂ (mg/L) | Temperature increase (°C) |
|---|---|
| 0.4 | 7.8 |
| 1 | 12.9 |
| 2.1 | 20.1 |

The utility of using an enclosure as the carrier 12 to enclose the chemical substance 14 is illustrated by Tables 6 and 7, which illustrate the increase of temperature detected by a fiberoptic temperature probe 16 with KI contained in an enclosure. For Table 6, the enclosure was PVC shrink tubing with holes. The holes were small enough to trap the KI powder but large enough to allow the diffusion of gas or vapor into the PVC tubing. For Table 7, the enclosure was gas-permeable Tyvek (RTM) tubing fabricated from heat-sealed 1073B Tyvek (RTM). The inner diameter of the enclosure was about 0.5 centimeters, and its length was approximately 1.5 centimeters. For Table 6, about 0.2 grams of KI powder was enclosed in the PVC tubing and the concentration monitor 10 was re-used for all measurements. For Table 7, about 0.2 grams of KI powder was enclosed in the Tyvek (RTM) pouch and the concentration monitor 10 was also re-used for all measurements. Table 1 test conditions were used for these measurements. It is apparent that measurable increases of temperature were detected for increasing H₂O₂ concentration when using both embodiments of a gas-permeable pouch as the carrier 12. The results also demonstrate that the concentration monitor 10 can be re-used and the measurements are reproducible.

**Table 6:**

| Concentration of H₂O₂ (mg/L) | Temperature increase (°C) | | |
|---|---|---|---|
| | Trial #1 | Trial #2 | Average |
| 0.2 | 1.1 | 1.1 | 1.1 |
| 0.4 | 9.5 | 8.8 | 9.2 |
| 1.0 | 13.6 | 13.6 | 13.6 |

**Table 7:**

| Concentration of H₂O₂ (mg/L) | Temperature increase (°C) | | |
|---|---|---|---|
| | Trial #1 | Trial #2 | Average |
| 0.4 | 9.7 | 8.4 | 9.1 |
| 1.0 | 17.3 | 16.8 | 17.1 |
| 1.4 | 23.6 | 23.6 | 23.6 |

A chemical substance 14 comprising a polymer comprising hydroxyl functional groups may also be used to fabricate a hydrogen peroxide monitor. For example, polyethylene glycol or PEG, with a formulation of H(OCH₂CH₂)ₙOH, mixed with an acrylic binder in aqueous suspension provides a hydrogen peroxide monitor compatible with the present invention. Such chemical substances have a high specificity to oxidative gas or vapor, such as H₂O₂, and essentially no sensitivity to H₂O. Persons skilled in the art appreciate that other polymers containing hydroxyl functional groups are also compatible with the present invention.

To examine the utility of a PEG/acrylic suspension, various H₂O₂ monitors were fabricated using the following procedure. A 1:1 ratio by weight PEG/acrylic mixture was made by mixing and stirring 5 g of acrylic binder (Vivitone, Inc., product number 37-14125-001, metallic binder LNG) with 5 g of PEG (Aldrich, Inc., product number 30902-8, molecular weight of approximately 10,000) in a 20-g scintillation vial. Other embodiments compatible with the present invention can utilize ratios other than 1:1. The mixture was then heated to approximately 75 °C and stirred thoroughly. After allowing the mixture to cool to room temperature, the vial containing the suspension was capped and stored in a cool, dark environment.

To fabricate each H₂O₂ monitor, the metal surface of a thermocouple was chemically treated to improve the adhesion of the chemical substance 14 to the carrier 12. The thermocouple was soaked in isopropyl alcohol for approximately two minutes and its end was brushed lightly to remove debris. After air-drying for approximately five minutes, the end of the thermocouple was soaked in approximately 10-20% by volume sulfuric acid (H₂SO₄) for approximately two minutes, then rinsed thoroughly in generous amounts of deionized water. The thermocouple was then dried in an oven at approximately 55 °C for approximately five minutes, then allowed to cool to room temperature outside the oven for approximately five minutes. The end of the thermocouple was then coated with the PEG/acrylic mixture by dipping the end of the thermocouple into the vial containing the mixture. Note that in order to produce a thicker overall coating, the end of the thermocouple can be dipped repeatedly. The thermocouple was then returned to the oven to dry at approximately 55 °C for approximately five minutes. A similar procedure was used to fabricate PEO/acrylic H₂O₂ monitors.

The above procedure can generate H₂O₂ monitors which are durable, inexpensive, and easy to manufacture. Also, PEG/acrylic mixtures have a relatively long shelf life of more than approximately three years. By utilizing a coating of the PEG/acrylic suspension, very small and flexible H₂O₂ monitors can be fabricated with different sizes and shapes. For example, if it is desirable to measure the H₂O₂ concentration within a narrow tube, the reactive chemical substance can be coated onto an optical fiber such as a Luxtron® fluoroptic temperature probe, a fiberoptic temperature probe, or on a metal wire of a thermistor or thermocouple assembly.

PEG/acrylic H₂O₂ monitors and PEO/acrylic H₂O₂ monitors fabricated by the above procedure were tested in a STERRAD® 100 low temperature, hydrogen peroxide gas plasma sterilization system. The sensitivity of these H₂O₂ monitors to hydrogen peroxide vapor is illustrated in Table 8 which provides the measured temperature increases in °C generated by the H₂O₂ monitors for different concentrations of H₂O₂ in the STERRAD® chamber. The change of temperature is referenced to the temperature read by the thermocouple just prior to the injection of H₂O₂.

**Table 8:**

| | Temperature Increase (°C) | |
|---|---|---|
| H₂O₂ (mg/L) | PEG/acrylic | PEO/acrylic |
| 0.41 | 2.6 | 2.0 |
| 0.77 | 3.4 | 3.5 |
| 1.45 | 5.8 | 5.6 |
| 2.87 | 9.4 | 9.7 |
| 5.73 | 16.1 | 14.0 |
| 11.5 | 24.2 | 22.0 |

Measured temperature increases for known H₂O₂ concentrations can be used to generate a calibration curve for such H₂O₂ monitors. The H₂O₂ responses of individual H₂O₂ monitors using the same chemical substance/carrier mixture were substantially similar to one another, indicating that H₂O₂ monitors with reproducible responses to H₂O₂ can be produced. For sufficient reproducibility among the H₂O₂ monitors using the same chemical substance/carrier mixture, a standard response equation can express the response for all such H₂O₂ monitors, thereby eliminating the need for individual calibration of the H₂O₂ monitors to convert the temperature change into a measurement of the H₂O₂ concentration.

H₂O₂ monitors compatible with the present invention with a reactive chemical substance/carrier such as the PEG/acrylic mixture can utilize other temperature probes 16 besides thermocouples. Appropriate temperature probes 16 include, but are not limited to, glass thermometers, thermocouples, thermistors, RTD probes, temperature strips, optical temperature sensors, and infrared temperature sensors. In addition, the sensing surface of the temperature probe 16 can be chemically or mechanically etched to improve the adhesion between the reactive chemical substance 14 and the temperature probe 16. The reactive chemical substance 14 can be coated onto the temperature sensitive surface of the temperature probe 16 by a variety of methods, including but not limited to, dipping, painting, spraying, chemical vapor deposition, or electrochemical plating. For faster response times, it is preferable to apply a thin coat of the reactive chemical substance 14 on the temperature probe 16 with low thermal mass. The thickness of the coating can also be controlled by adjusting the dwelling time or the speed of withdrawal of the probe 16 from the solution as it is being coated, and the viscosity of the reactive chemical substance 14. Additional layers of the reactive chemical substance 14 can be added to the initial coating to improve signal strength and/or sensitivity.

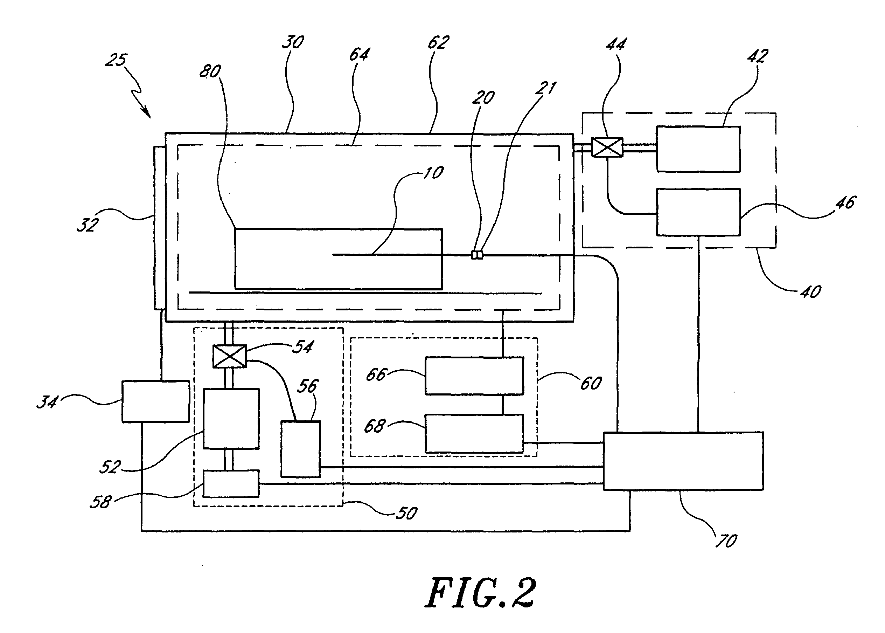

Figure 2 schematically illustrates a known sterilization system 25 compatible with embodiments of the present invention. The sterilization system 25 has a vacuum chamber 30 with a door 32 through which items to be sterilized are entered into and removed from the chamber 30. The door is operated by utilizing a door controller 34. The vacuum chamber 30 also has a gas inlet system 40, a gas outlet system 50, and a radio-frequency (rf) system 60. Other embodiments compatible with the present invention can utilize a low frequency plasma sterilization system, such as that described in "Sterilization System Employing Low Frequency Plasma", U.S. Patent 6,458,321. Comprising the gas inlet system 40 is a source of hydrogen peroxide (H₂O₂) 42, a valve 44, and a valve controller 46. The gas outlet system 50 comprises a vacuum pumping system 52, a valve 54, a valve controller 56, and a vacuum pumping system controller 58. In order to apply radio-frequency energy to the H₂O₂ in the vacuum chamber 30, the rf system 60 comprises a ground electrode 62, a powered electrode 64, a power source 66, and a power controller 68. The sterilization system 25 is operated by utilizing a control system 70 which receives input from the operator, and sends signals to the door controller 34, valve controllers 46 and 56, vacuum pumping system controller 58, and power controller 68. Coupled to the control system 70 (e.g., a microprocessor) is the concentration monitor 10, which sends signals to the control system 70 which are converted into information about the H₂O₂ concentration in the vacuum chamber 30 at the location of the concentration monitor 10. The sterilized article 80 is shown to be positioned in the chamber 30 with concentration monitor 10 located in the load region to monitor the concentration of hydrogen peroxide in the load region. Persons skilled in the art are able to select the appropriate devices to adequately practice the present invention.

The heat produced between the oxidative gas or vapor and the chemical substance 14 may not be the same for different configurations of the concentration monitor 10, carrier 12, and chemical substance 14. Therefore, for a given type of concentration monitor 10, a calibration curve needs to be established to determine the relationship between the concentration of oxidative gas or vapor and the heat produced. Once the calibration curve is established, the heat detected during the measurement can be converted to the concentration of the oxidative gas or vapor around the monitor 10.

By coupling the operation of the sterilization system 25 with the H₂O₂ concentration measured by the concentration monitor 10, the sterilization system 25 is assured of operating with an appropriate amount of H₂O₂ in the region of the articles to be sterilized. First, if the H₂O₂ concentration is determined to be too low for adequate sterilization, the control system 70 can signal the inlet valve controller 46 to open the inlet valve 44, thereby permitting more H₂O₂ into the chamber 30. Alternatively, if the H₂O₂ concentration is determined to be too high, the control system 70 can signal the outlet valve controller 56 to open the outlet valve 54, thereby permitting the vacuum pumping system 52 to remove some H₂O₂ from the chamber 30. Furthermore, if the sterilization system is being operated in a dynamic pumping mode (i.e., H₂O₂ is introduced into the chamber 30 via the inlet valve 44 while at the same time, it is pumped out via the outlet valve 54), then either the inlet valve 44 or the outlet valve 54, or both can be adjusted in response to the measured H₂O₂ concentration to ensure an appropriate level of H₂O₂.

Because the concentration monitor 10 provides localized information regarding the H₂O₂ concentration, it is important to correctly position the concentration monitor 10 within the sterilization chamber 30. In some preferred embodiments, the concentration monitor 10 is fixed to a particular position within the sterilization chamber 30 in proximity to the position of the sterilized articles 80. In other preferred embodiments, the concentration monitor 10 is not fixed to any particular position within the sterilization chamber 30, but is placed on or near the sterilized article 80 itself. In this way, the concentration monitor 10 can be used to measure the H₂O₂ concentration to which the sterilized article 80 is exposed. In particular, if the sterilized article 80 has a region which is exposed to a reduced concentration of H₂O₂ due to occlusion or a reduced opening, then the concentration monitor 10 can be placed within this region to ensure that a sufficient H₂O₂ concentration is maintained to sterilize this region. The small size of the concentration monitor of the present invention permits the concentration monitor to be placed in very restricted volumes, such as the inner volume of a lumen, or in a container or wrapped tray. In still other embodiments of the present invention, a plurality of concentration monitors 10 can be utilized to measure the H₂O₂ concentration at various positions of interest.

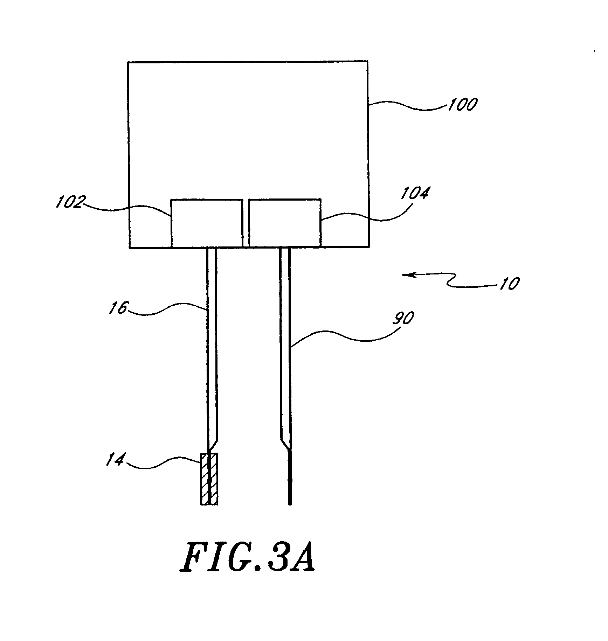

The temperature of the temperature probe 16 within the sterilization chamber 30 may fluctuate due to other factors unrelated to the hydrogen peroxide concentration. These non-H₂O₂-related temperature fluctuations may be misconstrued as resulting from changes of the H₂O₂ concentration in the sterilization chamber 30, and may result in measurement errors. In certain embodiments, as schematically illustrated in Figure 3A, a reference temperature probe 90 can be utilized in conjunction with the temperature probe 16 of the concentration monitor 10 to provide a measure of the ambient temperature within the sterilization chamber 30 to improve the performance of the concentration monitor 10.

The reference temperature probe 90 in proximity to the temperature probe 16 can then be used to measure the non-H₂O₂-related temperature fluctuations and compensate for these non-H₂O₂-related temperature fluctuations from the temperature reading of the temperature probe 16. In certain embodiments, the non-H₂O₂-related temperature fluctuations are monitored substantially simultaneously with the temperature readings of the temperature probe 16. Typically, the reference temperature probe 90 is substantially identical to the temperature probe 16, but does not comprise the reactive chemical substance 14. For example, a PEG/acrylic H₂O₂ concentration monitor 10 can comprise a reference temperature probe 90 with the acrylic binder but without the PEG polymer. Alternatively, the H₂O₂ concentration monitor 10 can comprise a bare reference temperature probe 90 without the binder or the reactive chemical substance 14.

In Figure 3A, the concentration monitor 10 comprises a reference temperature probe 90 and a temperature probe 16, the reference temperature probe 90 separate from the temperature probe 16. In known arrangement, the concentration monitor 10 comprises a microprocessor 100, and the temperature probe 16 and the reference temperature probe 90 are each coupled to a separate data acquisition channel 102, 104 of the microprocessor 100. The microprocessor 100 can comprise an algorithm, in hardware, software, or both, which subtracts the ambient temperature, as determined by the reference temperature probe 90, from the temperature detected by the temperature probe 16 to arrive at the temperature rise due to the oxidative gas or vapor concentration in the sterilization chamber 30. In such an arrangement, electrical connections between the temperature probe 16, reference temperature probe 90, and microprocessor 100 require two data acquisition channels which, in certain embodiments, are too large in size to allow the temperature probe 16 and reference temperature probe 90 to be placed in certain narrow lumens.

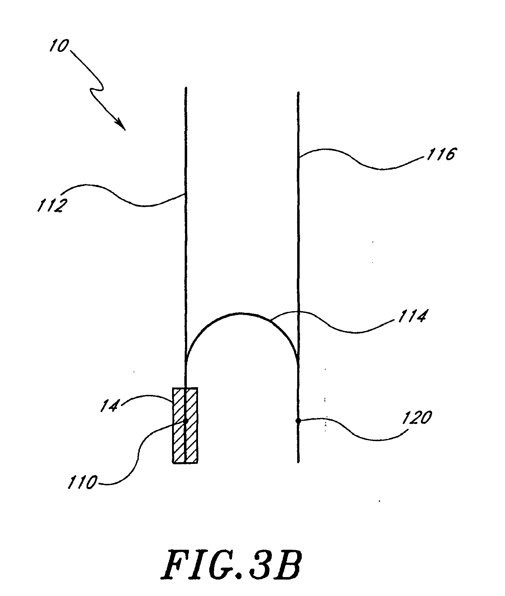

In certain embodiments of the invention, as schematically illustrated in Figure 3B, the concentration monitor 10 comprises a first thermocouple junction 110 and a chemical substance 14 coupled to the first thermocouple junction 110. The chemical substance 14 is reactive with the oxidative gas or vapor to produce heat. The first thermocouple junction 110 comprises a first conductor 112 and a second conductor 114 coupled to the first conductor 112, the second conductor 114 being different from the first conductor 112.

The concentration monitor 10 further comprises a second thermocouple junction 120 which, in certain embodiments, is substantially similar to the first thermocouple junction 110. The second thermocouple junction 120 is coupled in series to the first thermocouple junction 110. In certain embodiments, as schematically illustrated in Figure 3B, the second thermocouple junction 120 comprises a third conductor 116 and the second conductor 114, the third conductor 116 coupled to the second conductor 114. In embodiments in which the second thermocouple junction 120 is substantially similar to the first thermocouple junction 110, the third conductor 116 is substantially similar to the first conductor 112. For example, the first conductor 112 and third conductor 116 can comprise constantan (copper-nickel alloy) wire and the second conductor 114 can comprise iron wire, thereby forming two J-type thermocouple junctions in series. Typically, such thermocouple junctions have sensitivities on the order of µV/°C. The first and second thermocouple junctions 110, 120 are substantially thermally isolated from one another, but are placed in the same diffusion-restricted region as one another. As used herein, the term "diffusion-restricted region" refers to a region which is reached by the oxidative gas or vapor only after diffusing through such diffusion-limiting features as small openings, gas-permeable membranes, or along long, narrow diffusion paths, such as lumens.

Placed in an environment with no oxidative gas or vapor, the first thermocouple junction 110 and second thermocouple junction 120 each generates a voltage indicative of the ambient temperature. In embodiments in which the second thermocouple junction 120 is substantially similar to the first thermocouple junction 110, both thermocouple junctions 110, 120 generate the same voltage but are oriented to have opposite polarity such that the net voltage across both the first thermocouple junction 110 and the second thermocouple junction 120 is zero. Such a concentration monitor 10 in an environment with no oxidative gas or vapor responds to temperature fluctuations by maintaining a zero net voltage across the two thermocouple junctions 110, 120.

Upon exposing the chemical substance 14 to the oxidative gas or vapor, the heat generated by the chemical substance 14 increases the temperature of the first thermocouple junction 110 while the temperature of the second thermocouple junction 120 remains substantially unaffected, remaining at the ambient temperature. In embodiments in which the second thermocouple junction 120 is substantially similar to the first thermocouple junction 110, the voltage generated by the first thermocouple junction 110 is different from the voltage generated by the second thermocouple junction 120 in the presence of the oxidative gas or vapor. The net voltage across the first and second thermocouple junctions 110, 120 is responsive to the temperature difference between the first thermocouple junction 110 with the chemical substance 14 and the second thermocouple junction 120 without the chemical substance 14. Since any temperature fluctuations not due to the oxidative gas or vapor concentration affect both thermocouple junctions 110, 120 equally, the net voltage across both the first thermocouple junction 110 and second thermocouple junction 120 then corresponds to the concentration of the oxidative gas or vapor.

In certain embodiments, the first thermocouple junction 110 and second thermocouple junction 120 are each formed by welding together two conductors comprising different materials. Alternatively, one or both of the thermocouple junctions 110, 120 is formed by twisting together two conductors comprising different materials. Other embodiments compatible with the present invention can form the first and second thermocouple junctions 110, 120 by connecting the two conductors together using other methods. As schematically illustrated in Figures 3A and 3B, the conductors of certain embodiments are metal wires. The materials for the conductors which comprise the first thermocouple junction 110 and second thermocouple junction 120 are selected to provide thermocouple junctions with sufficient thermoelectric sensitivity and generally low cost, high electrical conductivity, low thermal conductivity, and good material compatibility with the sterilization process.

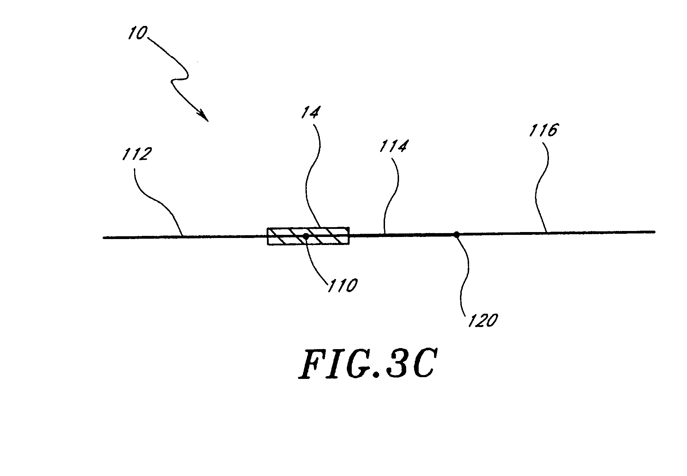

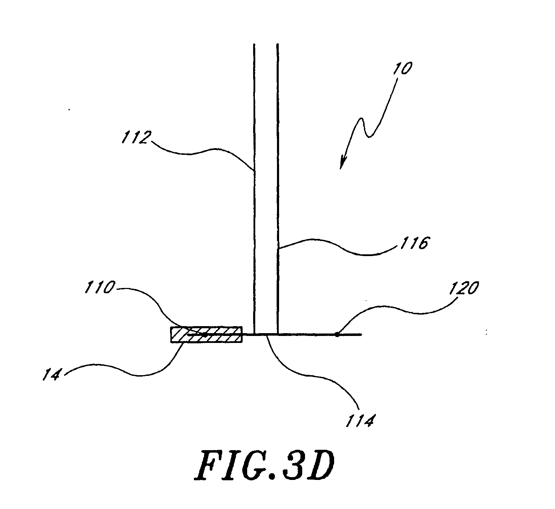

As schematically illustrated in Figure 3C, in certain embodiments, the concentration monitor 10 has a linear configuration and comprises a first thermocouple junction 110 and a second thermocouple junction 120. The first thermocouple junction 110 is formed by coupling a first conductor 112 to a second conductor 114 such that the first conductor 112 and second conductor 114 are substantially colinear. The second thermocouple junction 120 is formed by coupling the second conductor 114 to a third conductor 116 such that the second conductor 114 and third conductor 116 are also substantially colinear. The first thermocouple junction 110 is coupled to the chemical substance 14 and the second thermocouple junction 120 is not coupled to the chemical substance 14. Such an embodiment is especially useful for monitoring the concentration of the oxidative gas or vapor within a long, narrow lumen. Similarly, in the embodiment schematically illustrated in Figure 3D, the concentration monitor 10 has a "T" configuration. Other configurations are compatible with embodiments of the present invention, and the particular embodiment utilized can be designed for compatibility with the region in which the oxidative gas or vapor concentration is to be measured.

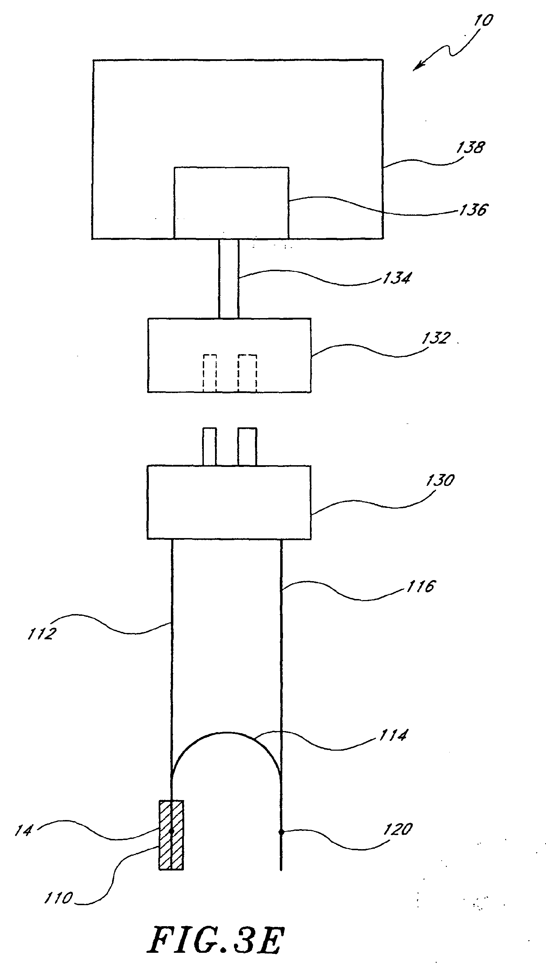

As schematically illustrated in Figure 3E, in certain embodiments, the concentration monitor 10 comprises a first connector 130, second connector 132, cable 134, data acquisition channel 136, and microprocessor 138. The first connector 130 and second connector 132 can be coupled together to electrically connect the first conductor 112 and third conductor 116 via the cable 134 to the data acquisition channel 136 of the microprocessor 138. The first connector 130 and second connector 132 can also be decoupled so that, for example, the concentration monitor 10 can be repositioned at a different location within the sterilization chamber.

The embodiments schematically illustrated in Figures 3B-3E provide advantages over the known arrangement schematically illustrated in Figure 3A. First, using two thermocouple junctions 110, 120 in series requires only one sensing circuit or one data acquisition channel to monitor the concentration of the oxidative gas or vapor, as opposed to two data acquisition channels as in Figure 3A. Besides providing a potential cost savings, using only one data acquisition channel or sensing circuit eliminates the potential effects of variations between the multiple channels or sensing circuits. Second, since the net voltage across the two thermocouple junctions 110, 120 represents a temperature difference rather than an absolute temperature, the dynamic range of values is smaller, so the an analog-to-digital converter with a given number of bits can thereby provide greater precision when used in the chemical concentration measuring system. Third, because only one pair of conductors is needed to detect the net voltage across the two thermocouple junctions 110, 120, the size of the concentration monitor 10 can be made smaller to fit into various diffusion-restricted environments, such as narrow lumens.

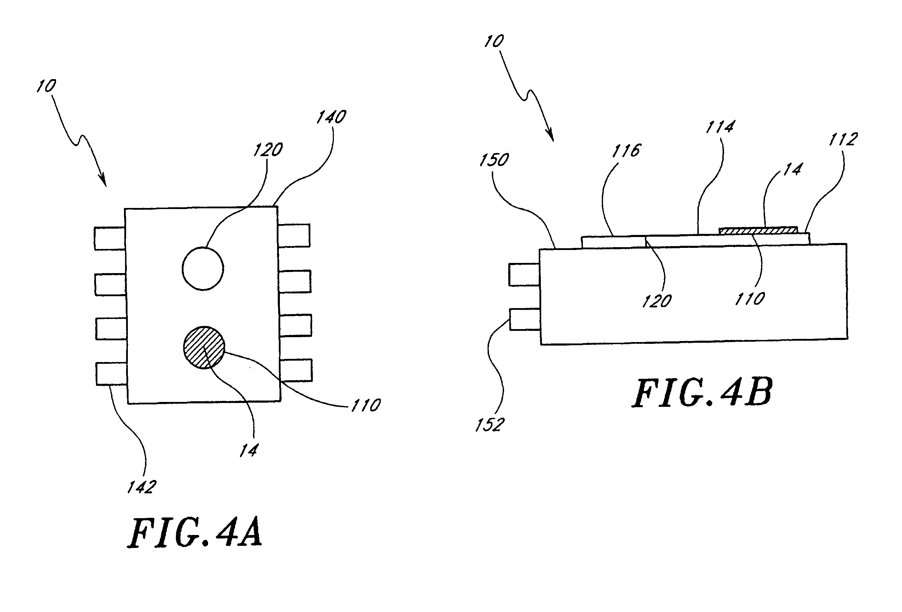

As schematically illustrated in Figure 4A, in certain embodiments, the concentration monitor 10 comprises an integrated circuit chip 140 which comprises circuitry which includes the first and second thermocouple junctions 110, 120, chemical substance 14, and a microprocessor or other sensing circuit (not shown). The integrated circuit chip 140 is configured to output a signal on one or more of its pins 142 to communicate the measured concentration to the rest of the chemical concentration measuring system. In certain embodiments, standard lithographic techniques can be used to fabricate the first and second thermocouple junctions 110, 120 by depositing and etching overlapping metal layers with different materials onto a substrate. Persons skilled in the art are able to fabricate such concentration monitors 10 in accordance with embodiments of the present invention.

As schematically illustrated in Figure 4B, in certain embodiments, the first and second thermocouple junctions 110, 120 are formed from a first conductor 112, second conductor 114, and third conductor 116, where one or more of the conductors comprises a thin conductive film configuration. The chemical substance 14 is coupled to the first thermocouple junction 110, and in certain embodiments, can also have a thin film configuration. In embodiments in which first and second thermocouple junctions 110, 120 formed by thin film conductors are part of a thin film concentration monitor 150, a signal indicative of the measured concentration can be provided on one or more of the pins 152. In certain embodiments, a thin film concentration monitor 150 may be incorporated into the packaging of the articles to be sterilized, thereby providing localized concentration information from a plurality of articles in the load.

In embodiments in which the first thermocouple junction 110 is substantially similar to the second thermocouple junction 120, further advantages are achieved. First, the concentration monitor 10 does not require an algorithm to correct for ambient temperature, since the net voltage across the two thermocouple junctions due to ambient temperature is null. Second, a cold junction compensation is not required, since ambient temperature has effectively no contribution. Third, only a relatively small amount of the second conductor 114 is needed to form the two thermocouple junctions, thereby realizing a cost savings over other embodiments.

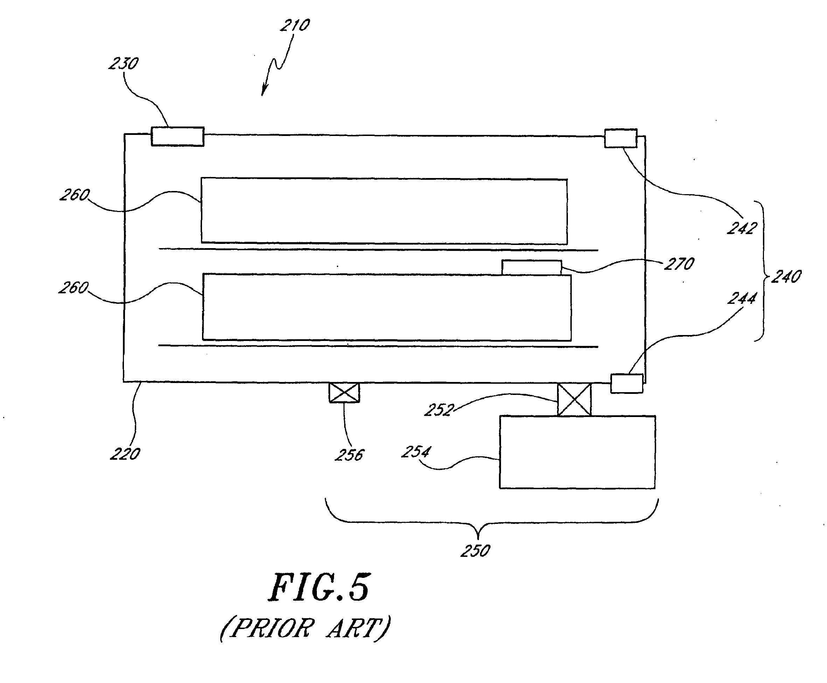

Figure 5 schematically illustrates a sterilization system 210 as disclosed in the prior art. Examples of such sterilization systems 210 are disclosed by Van Den Berg, et al. (U.S. Patent No. 5,847,393), Stewart, et al. (U.S. Patent No. 5,872,359), Goldenberg, et al. (U.S. Patent No. 6,061,141), and Prieve, et al. (U.S. Patent No. 6,269,680), which are incorporated in their entirety by reference herein. Other sterilization systems 210 are suitable for embodiments of the present invention, and the sterilization system 210 schematically illustrated in Figure 5 is not meant to be limiting to the present invention.

The sterilization system 210 comprises a sterilization chamber 220, a hydrogen peroxide source 230, a concentration monitor 240, and a vacuum system 250 comprising a valve 252, a pump 254, and a vent 256. The sterilization chamber 220 contains the load 260 to be sterilized and is sufficiently gas-tight to support a vacuum of approximately 300 mTorr or less. The sterilization system 210 also comprises a process controller (not shown) which transmits control signals to the source 230 and vacuum system 250 in response to user commands, system status, and hydrogen peroxide concentration as determined by the monitor 240. The sterilization system 210 can also comprise a plasma generating system (not shown).

The concentration monitor 240 is capable of measuring the concentration of hydrogen peroxide vapor in the sterilization chamber 220. Some prior art methods of measuring the concentration of hydrogen peroxide vapor include pressure measurement, dew point measurement, near-infrared absorption measurement, and ultraviolet absorption measurement. For example, as schematically illustrated in Figure 5, the concentration monitor 240 can comprise a ultraviolet light source 242 (e.g., a mercury vapor lamp) and an ultraviolet spectrometer 244. Ultraviolet light emitted from the light source 242 is transmitted through the vacuum to the spectrometer 244. Hydrogen peroxide vapor in the sterilization chamber 220 absorbs certain wavelengths of the ultraviolet light, and the amount of absorption is a function of the hydrogen peroxide concentration.

In such configurations, the concentration monitor 240 provides information regarding the average hydrogen peroxide concentration in the sterilization chamber 220. However, for loads 260 with diffusion-restricted regions (e.g., small crevices and long, narrow lumens), the concentration measurements by the monitor 240 do not always correlate with the hydrogen peroxide concentration in the diffusion-restricted regions. Besides the general problem of having the diffusion of hydrogen peroxide restricted by such constricted pathways, sterilization methods using an aqueous solution of hydrogen peroxide have certain other disadvantages. First, because water has a higher vapor pressure than does hydrogen peroxide, water vaporizes faster then does hydrogen peroxide from an aqueous solution. Second, water has a lower molecular weight than does hydrogen peroxide, so water diffuses faster than does hydrogen peroxide in the vapor state. Therefore, when an aqueous solution of hydrogen peroxide is vaporized in the area surrounding the load 260, the water vapor reaches the load 260 first and in higher concentrations. The water vapor therefore hinders or reduces the penetration of hydrogen peroxide vapor into the diffusion-restricted regions.

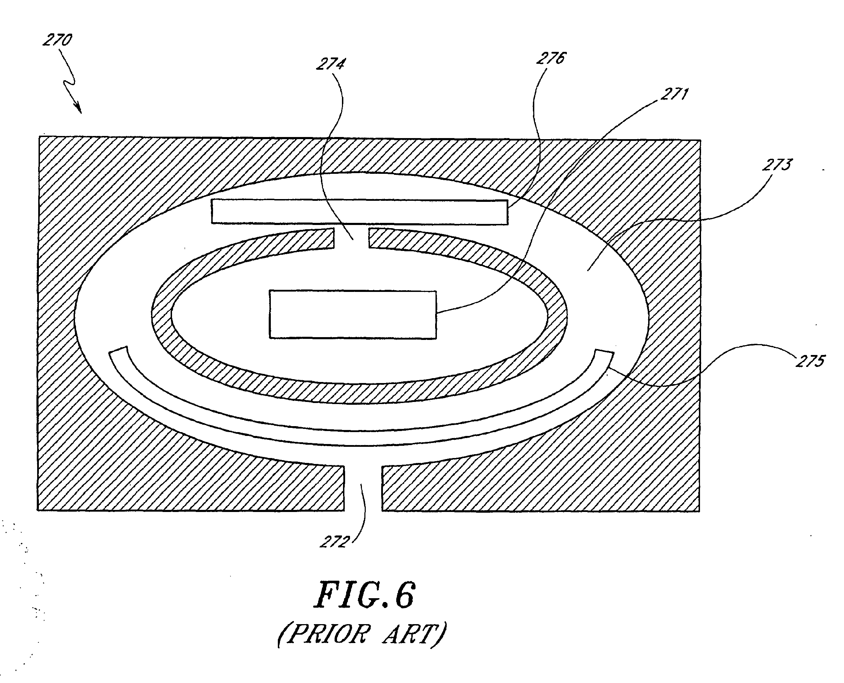

In an attempt to determine the hydrogen peroxide concentration in these diffusion-restricted regions of the load 260, a test pack 270 is typically introduced into the sterilization chamber 220 with the load 260. Figure 6 schematically illustrates one example of a test pack 270 as disclosed in the prior art. As described by Smith (U.S. Patent No. 5,552,320), which is incorporated in its entirety by reference herein, the test pack 270 comprises a biological indicator 271 in fluid communication with the surrounding atmosphere through an outer opening 272, an oval annular passage 273, and an inner opening 274. Within the oval annular passage 273 is a hydrogen peroxide absorber 275 positioned near the outer opening 272 which retards the passage of hydrogen peroxide through the oval annular passage 273. The test pack 270 also comprises a chemical indicator 276 which typically comprises a strip with a chemical which changes color when exposed to hydrogen peroxide. The chemical indicator 276 is positioned within the oval annular passage 273 near the inner opening 274 to provide a visual indication that the test pack 270 was exposed to hydrogen peroxide. Such a test pack 270 is available from Advanced Sterilization Products, Inc. of Irvine, CA (Ref. No. 14310).

The purpose of the test pack 270 is to impede access of the hydrogen peroxide to the biological indicator 271, thereby simulating the diffusion-restricted regions of the load 260. The dimensions of the various components of the test pack 270, such as the inner opening 274, outer opening 272, oval annular passage 273, and the hydrogen peroxide absorber 275, can be designed to mimic the diffusion of hydrogen peroxide to the diffusion-restricted regions of the load 260. This designing of the test pack 270 typically requires numerous sterilization trials in which a series of biological indicators 271 in various test packs 270 with different dimensions are compared to biological indicators in the diffusion-restricted region of the load 260. When the readings of the biological indicators in the test pack 270 and in the load 260 are in agreement, the test pack 270 provides a simulation of the diffusion-restricted region of the load 260.

In addition, when a load 260 of articles to be sterilized is placed in a sterilization system 210, some of the articles typically have less direct access to the hydrogen peroxide vapor than do others. To test the performance of the sterilization system 210 with respect to the articles having the least access to the hydrogen peroxide vapor, the test pack 270 is placed in a location which is anticipated to have a relatively low hydrogen peroxide concentration. In this way, the test pack 270 simulates the most difficult portions of the load 260 to be sterilized. If the biological indicator 271 of the test pack 270 is found to be sterilized, then the whole load 260 is also considered to be sterilized.

However, the biological indicator 271 of the test pack 270 provides information regarding the sterilization process only after the sterilization period has ended. Even more problematically, the results from the test pack 270 are typically only available after an incubation period. In addition, the test pack 270 is not reusable, since the packaging of the test pack 270 is torn apart in order to access and remove the biological indicator 271.

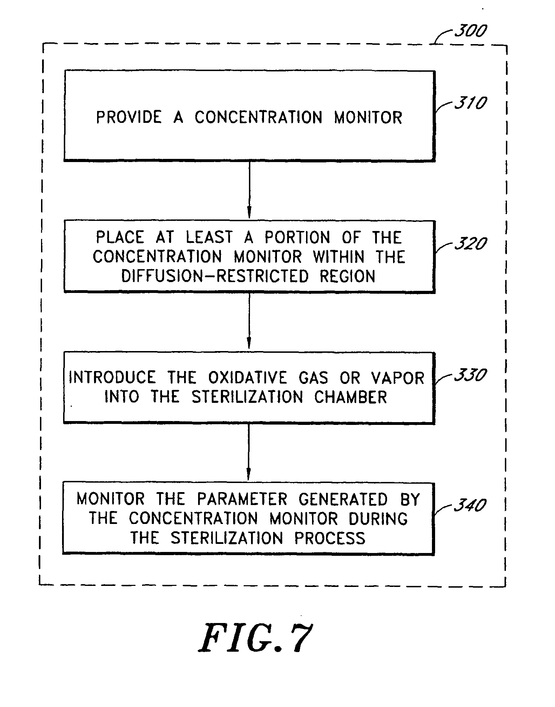

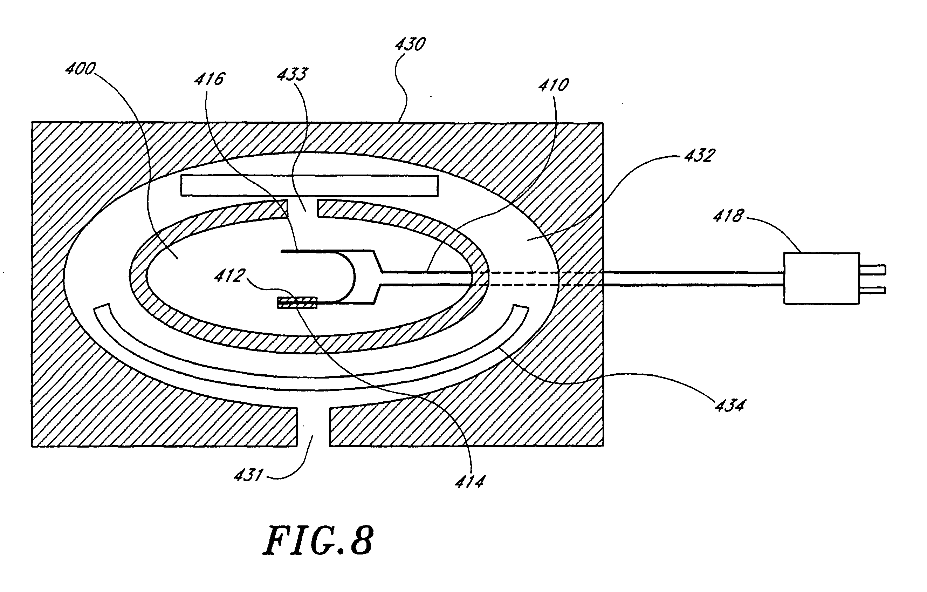

Figure 7 is a flow diagram of a method 300 in accordance with an embodiment of the present invention. During the sterilization process, the method enables the monitoring of a concentration of an oxidative gas or vapor in a diffusion-restricted region 400 in fluid communication with a sterilization chamber 220 during a sterilization process. The flow diagram is described with reference to Figure 8, which schematically illustrates a diffusion-restricted region 400 and concentration monitor 410 compatible with embodiments of the present invention. Persons skilled in the art are able to recognize that, while the flow diagram illustrates a particular embodiment with steps in a particular order, other embodiments with different orders of steps are also compatible with the present invention.

In an operational block 310, a concentration monitor 410 is provided. The concentration monitor 410 responds to the oxidative gas or vapor by generating a parameter. In certain embodiments, as schematically illustrated in Figure 8, the concentration monitor 410 comprises a first temperature sensing device 412 and a chemical substance 414 reactive with the oxidative gas or vapor to produce heat. The first temperature sensing device 412 is coupled to the chemical substance 414 and responds to the heat produced by the chemical substance 414 and the oxidative gas or vapor by generating a first signal. The parameter is generated in response to the first signal. As schematically illustrated in Figure 8, the first temperature sensing device of certain embodiments is a first thermocouple junction 412 and the first signal comprises a first voltage.

In other embodiments, the concentration monitor further comprises a second temperature sensing device 416 which generates a second signal, and the parameter is generated in further response to the second signal. In certain such embodiments, the second temperature sensing device 416 is a second thermocouple junction 416 which generates a second voltage. In still other embodiments, the second thermocouple junction 416 is coupled in series to the first thermocouple junction 412. A net voltage is generated across the first and second thermocouple junctions 412, 416 in response to the first and second voltages upon exposure of the chemical substance 414 to the oxidative gas or vapor, with the net voltage corresponding to the concentration of the oxidative gas or vapor.

In certain embodiments, the concentration monitor 410 also comprises an electrical connector 418 which facilitates connection and disconnection of the concentration monitor 410 with a chemical concentration monitoring system (not shown). In other embodiments, a concentration monitor 410 with a separate reference temperature probe may be used. In still other embodiments, a concentration monitor 410 may be used without any reference temperature probe. Persons skilled in the art recognize that other types of concentration monitors which provide a parameter corresponding to the concentration of the oxidative gas or vapor and which can have at least a portion placed within a diffusion-restricted region are compatible with embodiments of the present invention.

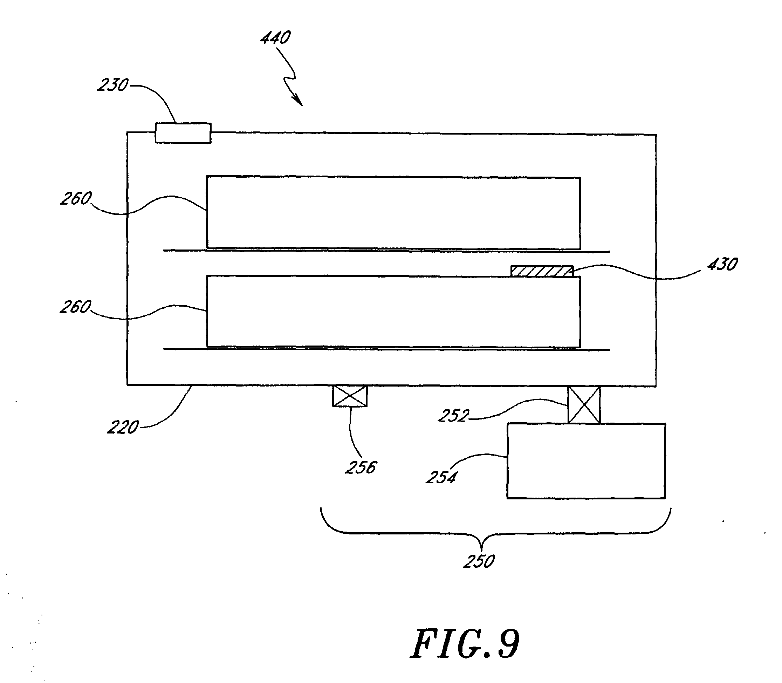

In an operational block 320, at least a portion of the concentration monitor 410 is placed within the diffusion-restricted region 400. As schematically illustrated in Figure 8, the portion of the concentration monitor 410 within the diffusion-restricted region 400 comprises the chemical substance 414. In certain embodiments, as schematically illustrated in Figure 8, the diffusion-restricted region 400 is part of a test pack 430 which includes an outer passage 431, oval annular passage 432, inner passage 433, and a hydrogen peroxide absorber 434. The test pack 430 is placed in a sterilization system 440 with the load 260, as schematically illustrated in Figure 9. However, instead of the test packs of the prior art which utilized a biological indicator, a test pack 430 compatible with embodiments of the present invention utilizes the concentration monitor 410. Also, as is described more fully below, because the concentration monitor 410 provides a real-time measurement of the concentration of the oxidative gas or vapor during the sterilization process, the test pack 430 may not require the chemical indicator as is found in the test packs of the prior art.

In an operational block 330, the oxidative gas or vapor is introduced into the sterilization chamber 220. Because the diffusion-restricted region 400 is in fluid communication with the sterilization chamber 220, the oxidative gas or vapor also reaches the portion of the concentration monitor 410 in the diffusion-restricted region 400 at a concentration to be determined.

In an operational block 340, the parameter generated by the concentration monitor 410 is monitored during the sterilization process. The parameter is indicative of the concentration of the oxidative gas or vapor within the diffusion-restricted region 400. In this way, the concentration of the oxidative gas or vapor within the diffusion-restricted region 400 is monitored during the sterilization process.

In certain embodiments in which the concentration monitor 410 comprises the first thermocouple junction 412 coupled to the chemical substance 414 and the second thermocouple junction 416 in series with the first thermocouple junction 412, as schematically illustrated in Figure 8, the parameter is generated by the concentration monitor 410 in response to the net voltage across the first and second thermocouple junctions 412, 416. This net voltage is a function of the temperature difference between the first and second thermocouple junctions 412, 416. This temperature difference is the result of the reaction of the chemical substance 414 with the oxidative gas or vapor, which produces heat detected by the first thermocouple junction 412 but not by the second thermocouple junction 416. The amount of heat produced by the chemical substance 414 is correlated with the concentration of the oxidative gas or vapor.

In certain embodiments, monitoring 340 the parameter further comprises converting the parameter generated by the concentration monitor 410 to a measurement of the concentration of the oxidative gas or vapor in the diffusion-restricted region 400. In certain embodiments in which the concentration monitor 410 schematically illustrated in Figure 8 is used, the conversion of the parameter based on the measured net voltages across the first and second thermocouple junctions 412, 416 to concentration measurements typically requires a calibration table. Such calibration tables can be produced by exposing a concentration monitor 410 to known concentrations of the oxidative gas or vapor and noting the parameter based on the net voltage across the first and second thermocouple junctions 412, 416 for each known concentration. In this way, a real-time measurement of the concentration of the oxidative gas or vapor in the diffusion-restricted region 400 can be provided.

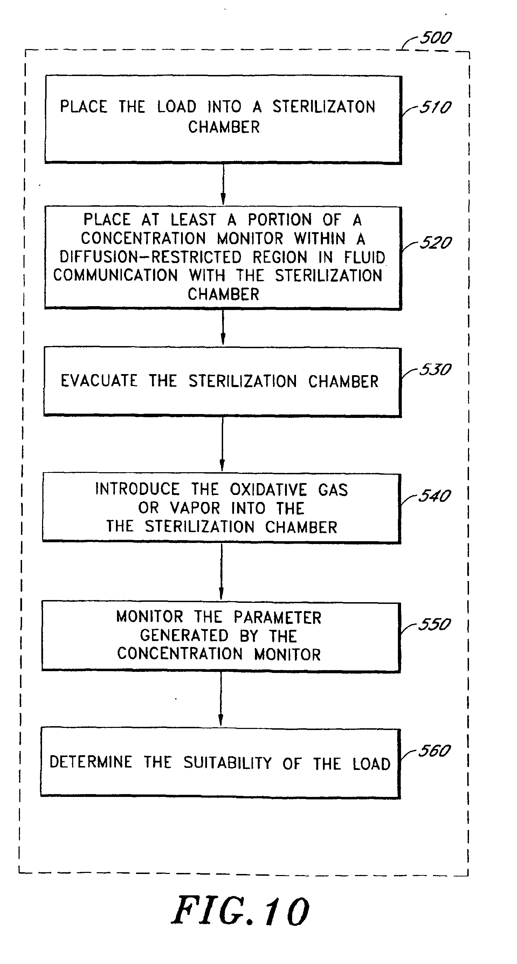

Figure 10 is a flow diagram of a method 500 of determining a suitability of a load 260 for sterilization with an oxidative gas or vapor during a sterilization process in accordance with another embodiment of the present invention. In an operational block 510, the load 260 is placed into the sterilization chamber 220 and in an operational block 520, at least a portion of a concentration monitor 410 is placed within a diffusion-restricted region 400 in fluid communication with the sterilization chamber 220. The concentration monitor 410 responds to the oxidative gas or vapor by generating a parameter corresponding to a concentration of the oxidative gas or vapor. As described above, Figure 8 schematically illustrates a concentration monitor 410 and a diffusion-restricted region 400 compatible with this embodiment of the present invention.

In an operational block 530, the sterilization chamber 220 is evacuated and in an operational block 540, the oxidative gas or vapor is introduced into the sterilization chamber 220. In this way, the load 260 is contacted by the oxidative gas or vapor. Because the diffusion-restricted region 400 is in fluid communication with the sterilization chamber 220, the concentration monitor 410 is exposed to the oxidative gas or vapor.

In an operational block 550, the parameter generated by the concentration monitor 410 is monitored. As described above, the parameter is indicative of the concentration of the oxidative gas or vapor within the diffusion-restricted region 400. In an operational block 560, the suitability of the load 260 is determined from the parameter indicative of the concentration of the oxidative gas or vapor within the diffusion-restricted region 400.

Typically, a load 260 is deemed suitable for use if the sterilization process is expected to have adequately sterilized the load 260. In prior art systems, the suitability of the load 260 is determined by an examination of a biological indicator 271 within a test pack 270 which mimics a diffusion-restricted region within the load 260. If the biological indicator 271 yields less than a predetermined number of viable microorganisms after being exposed to the sterilization process, the load 260 is deemed to be suitable for use. As described above, this prior art procedure results in a determination of the suitability of the load 260 only after the completion of the incubation period, which can be days after performing the sterilization process.

Conversely, using a concentration monitor 410 in accordance with embodiments of the present invention can determine the suitability of the load 260 during the sterilization process and avoid this problematic time delay. By noting the results from biological indicators in the diffusion-restricted region 400 as a function of the concentrations of the oxidative gas or vapor as measured by the concentration monitor 410 in the diffusion-restricted region 400, the correlation between the concentration parameter and the success of the sterilization process can be determined. Once this correlation is known, then the concentration readings from the concentration monitor 410 can be used to determine the success of future sterilization processes and the suitability of future loads 260. Sterilized articles from the load 260 can then be released for use as soon as the parameters during the sterilization process are known to have fallen within acceptable ranges. Release of articles in this way on the basis of parameters, such as the concentration readings from the concentration monitor 410, is termed parametric release of the load 260. Systems which can utilize parametric release of articles, rather than systems which utilize biological indicators, provide the advantages of quicker turnaround times, reduced costs, and less handling of the articles thereby reducing the possibility of subsequent contamination.

Embodiments of the present invention can define the threshold level of exposure to the oxidative gas or vapor which corresponds to a successful sterilization process in various ways. In certain embodiments, a successful sterilization process (i.e., one which produces a suitable load) is defined as one which achieved a minimum concentration level of the oxidative gas or vapor. In such embodiments, if the concentration monitor 410 indicates that the diffusion-restricted region has been exposed to at least this minimum concentration level during the sterilization process, the load is deemed to be suitable and is released. Alternatively in other embodiments, the success of the sterilization procedure is determined by the rate of change, if any, of the measured concentration of the oxidative gas or vapor during the sterilization process as measured by the concentration monitor 410. In certain such embodiments, if the diffusion-restricted region is exposed to a measured concentration with a rate of decrease which does not exceed a maximum value, the load is deemed to be suitable and is released. In other such embodiments, if the diffusion-restricted region is exposed to a measured concentration with a rate of increase which is not lower than a minimum value, the load is deemed to be suitable and is released. And in still other embodiments, the time-integrated measured concentration (i.e., the area under a plot of the measured concentration over the course of the sterilization process) in the diffusion-restricted region is used, such that the load is deemed to be suitable and is released upon exposing the diffusion-restricted region to at least a minimum time-integrated concentration. Other embodiments of the present invention can utilize other definitions of the success of a sterilization process which are determined by the concentration of the oxidative gas or vapor in the diffusion-restricted region as determined by the concentration monitor 410.

If the load is determined to be suitable, the sterilization process can be allowed to continue. If the load is determined to be not suitable, in certain embodiments, the sterilization process is aborted. Alternatively, upon determining that the load is not suitable, certain other embodiments introduce additional oxidative gas or vapor into the sterilization chamber 220. In still other embodiments, a determination that the load is not suitable activates an alarm to notify the user of this condition. Such embodiments can include a control feedback mechanism to control the process parameters.

Using a concentration monitor 410 in a diffusion-restricted region 400, such as in a test pack 430, provides additional advantages over the prior art methods which use biological indicators. First, the concentration monitor 410 can monitor the concentration of the oxidative gas or vapor in the diffusion-restricted region 400 at various times during the sterilization process. By controlling the vacuum system and the source of the oxidative gas or vapor in response to the measured concentration in the diffusion-restricted region 400 during the sterilization process, the sterilization system 440 can potentially actively maintain desired concentration levels throughout the sterilization process. Second, the concentration monitor 410 is reusable over many sterilization cycles, as compared to the one-time use of biological indicators, thereby realizing a cost savings.

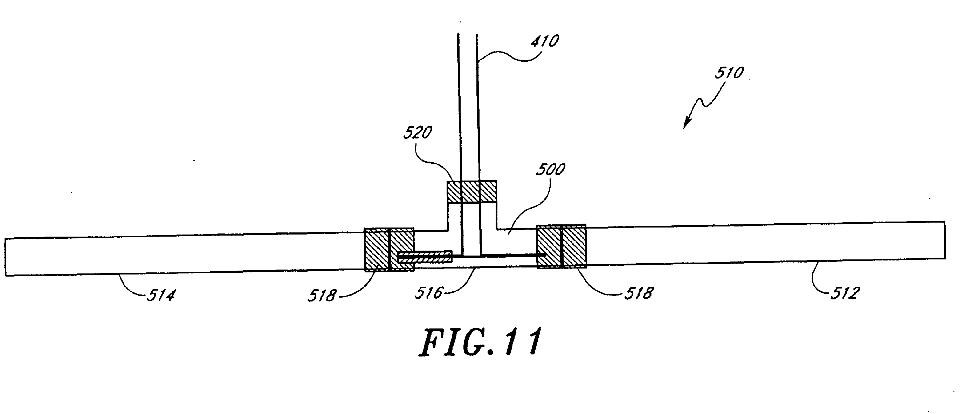

In certain embodiments, the concentration monitor 410 is advantageously placed in other diffusion-restricted regions besides the diffusion-restricted region 400 of a test pack 430. As schematically illustrated in Figure 11, in certain embodiments, the diffusion-restricted region comprises a region 500 inside a lumen 510. The lumen 510 of certain embodiments comprises a first tube 512 and a second tube 514 both coupled to a T-connector 516 containing a portion of the concentration monitor 410. The first tube 512, second tube 514, and T-connector 516 are coupled together by a pair of latex tubing connectors 518, thereby forming the lumen 510. The concentration monitor 410 is coupled to the T-connector 516 via a non-conductive epoxy 520 which seals closed the portion of the T-connector 516 through which the concentration monitor 410 extends. The first tube 512, second tube 514, and T-connector 516 are in fluid communication with one another, as well as with the atmosphere within the sterilization chamber 220. In certain embodiments, the dimensions of the first tube 512, second tube 514, and T-connector 516 are designed to mimic the dimensions of lumens within the load 260 to be sterilized.

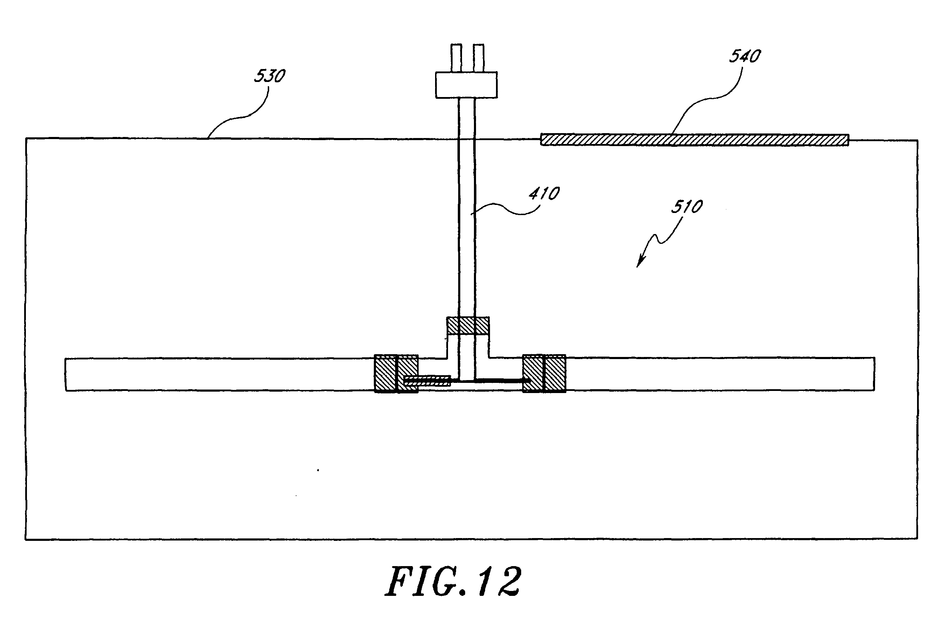

As schematically illustrated in Figure 12, in certain embodiments, the lumen 510 is placed inside a container 530 which is in fluid communication with the sterilization chamber 220. In certain embodiments, the container 530 comprises one or more openings 540 which are uncovered or, in alternative embodiments, comprise a gas-permeable material. An example of such an embodiment has the lumen 510 placed inside a sterilization tray wrapped in CSR wrap.

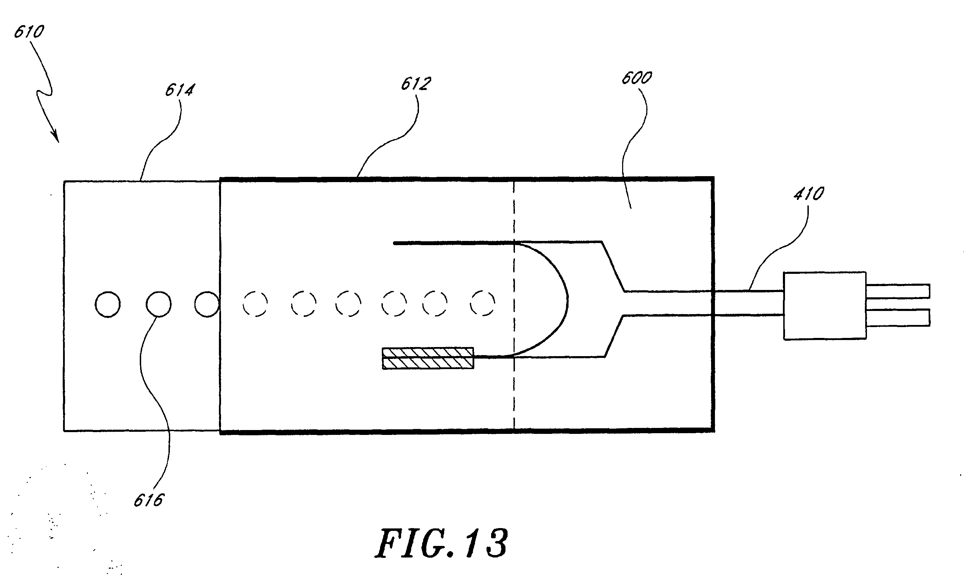

As schematically illustrated in Figure 13, in certain embodiments, the diffusion-restricted region comprises a region 600 inside a process challenge device (PCD) 610. In certain embodiments, the PCD 610 comprises an outer cylinder 612 and an inner cylinder 614 slidably coupled to the outer cylinder 612 and defining the inside region 600. The inner cylinder 614 comprises at least one opening 616. In the embodiment schematically illustrated in Figure 13, the inner cylinder 614 comprises a plurality of openings 616. The inner cylinder 614 can be positioned so that a fraction of the openings 616 is blocked by the outer cylinder 612 and a second fraction of the openings 616 is unblocked and provides fluid communication between the inside region 600 of the PCD and the sterilization chamber 220. The inner cylinder 614 can be slid to various positions to vary the fraction of the openings 616 which is blocked by the outer cylinder 612, thereby varying the diffusion path between the inside region 600 and the sterilization chamber 220. In this way, the PCD 610 can be tailored to mimic a diffusion-restricted region within the load 260, such as a packaged device.

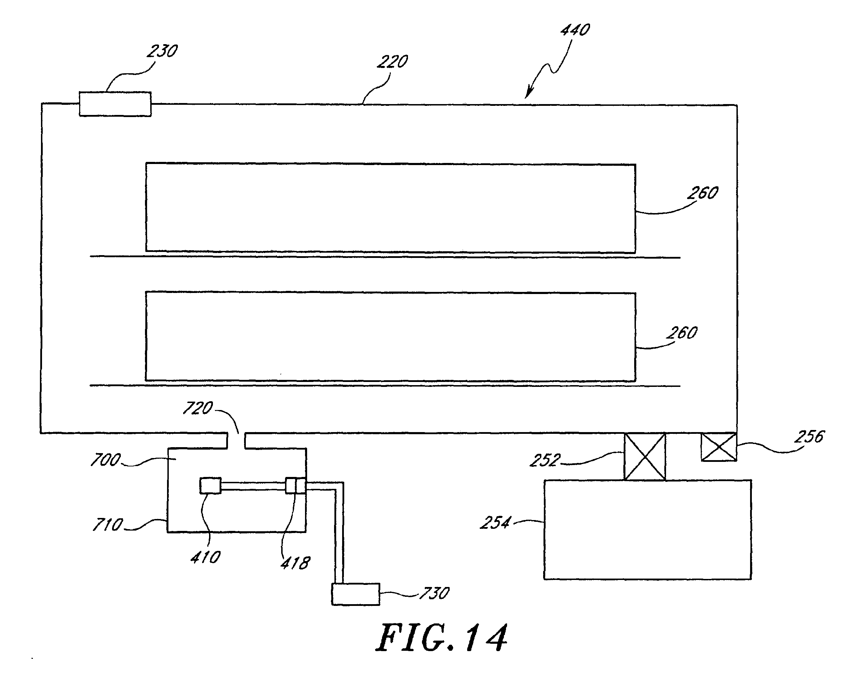

As schematically illustrated in Figure 14, in certain embodiments, the diffusion-restricted region comprises a region 700 inside a second chamber 710 in fluid communication with the sterilization chamber 220. A conduit 720 provides the fluid communication between the sterilization chamber 220 and the second chamber 710. In certain embodiments, the dimensions of the conduit 720 are designed so that the diffusion of the oxidative gas or vapor to the region 700 mimics the diffusion to the diffusion-restricted region within the load 260. Alternatively, the dimensions of the conduit 720 are designed to not appreciably affect the diffusion of the oxidative gas or vapor and the concentration monitor 410 is placed in a PCD 610 or a test pack 430 which mimics the diffusion-restricted region within the load 260.

The concentration monitor 410 of certain embodiments comprises a connector 418 which facilitates electrical connection and disconnection of the concentration monitor 410 with a chemical concentration measuring system 730. Embodiments comprising the second chamber 710 can provide easy access to the concentration monitor 410.

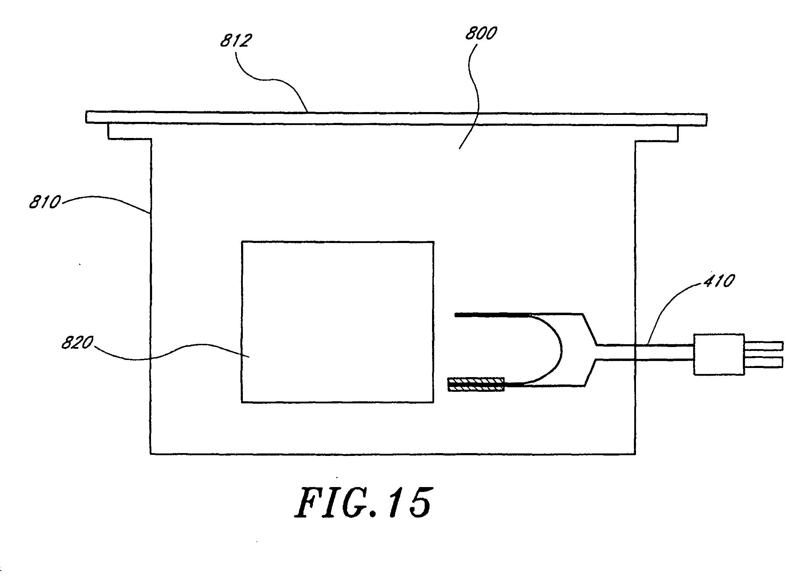

As schematically illustrated in Figure 15, in certain embodiments, the diffusion-restricted region comprises a region 800 inside the load 260. In certain such embodiments, the region 800 is inside a package 810 containing a device 820 to be sterilized. Each package 810 comprises a gas-permeable portion 812 so that the device 820 can be packaged, then sterilized, and the sterilized packaged device 820 can be shipped out to customers. As schematically illustrated in Figure 15, the package 810 can comprise a concentration monitor 410 which measures the concentration of the oxidative gas or vapor within the region 800 occupied by the device 820 to be sterilized. In embodiments which utilize a concentration monitor 410 which comprises thermocouple thin conductive films, the thin conductive films can be incorporated as part of the package 810. By using a concentration monitor 410 in conjunction with the devices 820 to be sterilized, information can be obtained regarding the exposure of the devices 820 to the oxidative gas or vapor during the sterilization of the load 260 and can be used to provide an evaluation of the sterilization process particularized to individual devices 820.

Various embodiments of the present invention have been described above. Although this invention has been described with reference to these specific embodiments, the descriptions are intended to be illustrative of the invention and are not intended to be limiting. Various modifications and applications may occur to those skilled in the art without departing from the scope of the invention as defined in the appended claims.

## Claims

1. A method of monitoring a concentration of an oxidative gas or vapor in a diffusion-restricted region (400) in fluid communication with a sterilization chamber (220) during a sterilization process, the method comprising:
providing a concentration monitor (10) responsive to the oxidative gas or vapor by generating a parameter, the concentration monitor comprising a first temperature sensing device, a second temperature sensing device and a chemical substance (14) reactive with the oxidative gas or vapor to produce heat, the first temperature sensing device coupled to the chemical substance and responsive to the heat produced by the chemical substance and the oxidative gas or vapor by generating a first signal, the second temperature sensing device generating a second signal, the parameter generated in response to the first signal and in further response to the second signal;
placing (520) at least a portion of the concentration monitor comprising the chemical substance (14) within the diffusion-restricted region (400);
introducing (540) the oxidative gas or vapor into the sterilization chamber (220); and
monitoring (550) the parameter generated by the concentration monitor during the sterilization process, thereby monitoring the concentration of the oxidative gas or vapor within the diffusion-restricted region (400) during the sterilization process,
wherein the first temperature sensing device comprises a first thermocouple junction (110) and the first signal comprises a first voltage, and
wherein the second temperature sensing device comprises a second thermocouple junction (120) which generates a second voltage,
**characterised in that** the second thermocouple junction (120) is coupled in series to the first thermocouple junction (110), and the parameter is generated in further response to a net voltage generated across the first and second thermocouple junctions in response to the first and second voltages.

2. The method of Claim 1, wherein monitoring the parameter further comprises converting the parameter generated by the concentration monitor (10) to a measurement of the concentration of the oxidative gas or vapor in the diffusion-restricted region (400).

3. The method of Claim 1, wherein the oxidative gas or vapor comprises hydrogen peroxide.

4. The method of Claim 1, wherein the diffusion-restricted region (400) comprises a region inside a test pack (430).

5. The method of Claim 1, wherein the diffusion-restricted region comprises a region (500) inside a lumen (510).

6. The method of Claim 5, wherein the lumen (530) is inside a container in fluid communication with the sterilization chamber (220).

7. The method of Claim 1, wherein the diffusion-restricted region comprises a region (600) inside a process challenge device (PCD) (610).

8. The method of Claim 7, wherein the PCD (610) is adjustable to vary a diffusion path between the region inside the PCD and the sterilization chamber (220) and the method further comprises adjusting the PCD to mimic a second diffusion-restricted region within the load (260).

9. The method of Claim 1, wherein the diffusion-restricted region comprises a region (700) inside a second chamber (710) in fluid communication with the sterilization chamber (220).

10. The method of Claim 1, wherein the diffusion-restricted region (400) is designed so as to mimic the diffusion of the oxidative gas or vapor to a second diffusion-restricted region of the load (260).

11. The method of Claim 1, wherein the diffusion-restricted region comprises a region (800) inside a packaged device (820).

12. The method of Claim 1, further comprising placing (510) a load (260) into the sterilization chamber (220) and determining (560) a suitability of the load.

13. The method of Claim 12, wherein the concentration monitor (10) is coupled to a control feedback mechanism which controls a process parameter of the sterilization system, and the method further comprises controlling the process parameter in response upon determining the suitability of the load (260).

14. The method of Claim 12, further comprising aborting the sterilization process upon determining that the load (260) is not suitable.

15. The method of Claim 12, further comprising introducing additional oxidative gas or vapor into the sterilization chamber (220) upon determining that the load (260) is not suitable.

16. The method of Claim 12, further comprising activating an alarm upon determining that the load (260) is not suitable.

17. The method of Claim 12, wherein determining (560) the suitability of the load comprises defining a minimum concentration level of the oxidative gas or vapor in the diffusion-restricted region (400) corresponding to a suitable load.

18. The method of Claim 12, wherein determining (560) the suitability of the load comprises defining a maximum rate of decrease of the concentration of the oxidative gas or vapor in the diffusion-restricted region (400) corresponding to a suitable load.

19. The method of Claim 12, wherein determining (560) the suitability of the load comprises defining a minimum rate of increase of the concentration of the oxidative gas or vapor in the diffusion-restricted region (400) corresponding to a suitable load.

20. The method of Claim 12, wherein determining (560) the suitability of the load comprises defining a minimum time-integrated concentration of the oxidative gas or vapor in the diffusion-restricted region (400) corresponding to a suitable load.

21. An apparatus for monitoring a concentration of an oxidative gas or vapor in a sterilization chamber (220) during a sterilization process, the apparatus comprising:
a diffusion-restricted region (400) in fluid communication with the sterilization chamber (220); and
a concentration monitor (10) responsive to the oxidative gas or vapor by generating a parameter, at least a portion of the concentration monitor within the diffusion-restricted region (400); the concentration monitor comprising a first temperature sensing device, a second temperature sensing device and a chemical substance (14) reactive with the oxidative gas or vapor to produce heat, the first temperature sensing device coupled to the chemical substance and responsive to the heat produced by the chemical substance and the oxidative gas or vapor by generating a first signal, the second temperature sensing device adapted to generate a second signal, the parameter generated in response to the first signal and in further response to the second signal,
wherein the first temperature sensing device comprises a first thermocouple junction (110) and the first signal comprises a first voltage, and
wherein the second temperature sensing device comprises a second thermocouple junction (120) and the second signal comprises a second voltage,
**characterised in that** the second thermocouple junction (120) is coupled in series to the first thermocouple junction (110), and the parameter is generated in further response to a net voltage generated across the first and second thermocouple junctions in response to the first and second voltages.

## Patentansprüche

1. Verfahren zur Überwachung einer Konzentration eines oxidativen Gases oder Dampfes in einem Bereich mit eingeschränkter Diffusion (400) in Fluidkommunikation mit einer Sterilisationskammer (220) während eines Sterilisationsprozesses, wobei das Verfahren umfaßt:
- Bereitstellung eines Konzentrationsüberwachungsgerätes (10), das durch die Erzeugung eines Parameters auf oxidatives Gas oder Dampf reagiert, wobei das Konzentrationsüberwachungsgerät ein erstes Temperaturmeßgerät, ein zweites Temperaturmeßgerät und eine chemische gekoppelt (14) umfaßt, die mit dem oxidativen Gas oder Dampf reagiert, um Wärme zu erzeugen, wobei das erste Temperaturmeßgerät mit der chemischen Substanz gekoppelt ist und auf die von der chemischen Substanz und dem oxidativen Gas oder Dampf erzeugte Wärme durch die Erzeugung eines ersten Signals reagiert, und das zweite Temperaturmeßgerät ein zweites Signal erzeugt, wobei der Parameter als Reaktion auf das erste Signal und als weitere Reaktion auf das zweite Signal erzeugt wird;
- Unterbringung (520) mindestens eines Teils des die chemische Substanz (14) enthaltenen Konzentrationsüberwachungsgerätes in dem Bereich mit eingeschränkter Diffusion (400);
- Einleitung (540) des oxidativen Gases oder Dampfes in die Sterilisationskammer (220); und
- Überwachung (550) des durch das Konzentrationsüberwachungsgerät erzeugten Parameters während des Sterilisationsprozesses, wodurch die Konzentration des oxidativen Gases oder Dampfes im Bereich mit eingeschränkter Diffusion (400) während des Sterilisationsprozesses überwacht wird,
- wobei das erste Temperaturmeßgerät eine erste Thermoelementverbindung (110) umfaßt und das erste Signal eine erste Spannung umfasst, und
- wobei das zweite Temperaturmeßgerät eine zweite Thermoelementverbindung (120) aufweist, die eine zweite Spannung erzeugt,
**dadurch gekennzeichnet, daß** die zweite Thermoelementverbindung (120) mit der ersten Thermoelementverbindung (110) in Reihe gekoppelt ist und der Parameter als weitere Reaktion auf eine Nettospannung erzeugt wird, die über der ersten und zweiten Thermoelementverbindung als Reaktion auf die erste und zweite Spannung erzeugt wird.

2. Verfahren nach Anspruch 1, **dadurch gekennzeichnet, daß** das Überwachen des Parameters weiterhin die Umstellung des durch das Konzentrationsüberwachungsgerät (10) erzeugten Parameters auf eine Messung der Konzentration des oxidativen Gases oder Dampfes in dem Bereich mit eingeschränkter Diffusion (400) umfaßt.

3. Verfahren nach Anspruch 1, **dadurch gekennzeichnet, daß** das oxidative Gas oder Dampf Wasserstoffperoxid enthält.

4. Verfahren nach Anspruch 1, **dadurch gekennzeichnet, daß** der Bereich mit eingeschränkter Diffusion (400) einen Bereich innerhalb einer Probepackung (430) umfaßt.

5. Verfahren nach Anspruch 1, **dadurch gekennzeichnet, daß** der Bereich mit eingeschränkter Diffusion einen Bereich (500) innerhalb eines Lumens (510) umfaßt.

6. Verfahren nach Anspruch 5, **dadurch gekennzeichnet, daß** das Lumen (530) innerhalb eines Behälters in Fluidkommunikation mit der Sterilisationskammer (220) steht.

7. Verfahren nach Anspruch 1, **dadurch gekennzeichnet, daß** der Bereich mit eingeschränkter Diffusion einen Bereich (600) innerhalb einer Prozeßabfragevorrichtung (PCD) umfaßt.

8. Verfahren nach Anspruch 7, **dadurch gekennzeichnet, daß** die PCD (610) einstellbar ist, um eine Diffusionsstrecke zwischen dem Bereich innerhalb der PCD und der Sterilisationskammer (220) zu variieren, und das Verfahren weiterhin die Einstellung der PCD umfaßt, um einen zweiten diffusionseingeschränkten Bereich innerhalb der Probe (260) zu imitieren.

9. Verfahren nach Anspruch 1, **dadurch gekennzeichnet, daß** der Bereich mit eingeschränkter Diffusion einen Bereich (700) innerhalb einer zweiten Kammer (710) in Fluidkommunikation mit der Sterilisationskammer (220) umfaßt.

10. Verfahren nach Anspruch 1, **dadurch gekennzeichnet, daß** der Bereich mit eingeschränkter Diffusion (400) so ausgeführt ist, um die Diffusion des oxidativen Gases oder Dampfes zu einem zweiten Bereich mit eingeschränkter Diffusion der Probe (260) zu imitieren.

11. Verfahren nach Anspruch 1, **dadurch gekennzeichnet, daß** der Bereich mit eingeschränkter Diffusion einen Bereich (800) innerhalb einer verpackten Vorrichtung (820) umfaßt.

12. Verfahren nach Anspruch 1, **dadurch gekennzeichnet, daß** es weiterhin die Unterbringung (510) einer Probe (260) in der Sterilisationskammer (220) und die Feststellung (560) der Eignung der Probe umfaßt.

13. Verfahren nach Anspruch 12, **dadurch gekennzeichnet, daß** das Konzentrationsüberwachungsgerät (10) an einen Kontrollrückkopplungsmechanismus angekoppelt ist, der einen Prozeßparameter des Sterilisationssystems kontrolliert, und daß das Verfahren weiterhin die Kontrolle des Prozeßparameters als Reaktion auf die Feststellung der Eignung der Probe (260) umfaßt.

14. Verfahren nach Anspruch 12, **dadurch gekennzeichnet, daß** es weiterhin den Abbruch des Sterilisationsprozesses umfaßt, wenn festgestellt wird, daß die Probe (260) nicht geeignet ist.

15. Verfahren nach Anspruch 12, **dadurch gekennzeichnet, daß** es weiterhin die Einleitung zusätzlichen oxidativen Gases oder Dampfes in die Sterilisationskammer (220) umfaßt, wenn festgestellt wird, daß die Probe (260) nicht geeignet ist.

16. Verfahren nach Anspruch 12, **dadurch gekennzeichnet, daß** es weiterhin die Auslösung eines Alarms umfaßt, wenn festgestellt wird, daß die Probe (260) nicht geeignet ist.

17. Verfahren nach Anspruch 12, **dadurch gekennzeichnet, daß** die Feststellung (560) der Eignung der Probe die Bestimmung eines minimalen Konzentrationsniveaus des oxidativen Gases oder Dampfes in dem Bereich mit eingeschränkter Diffusion (400) umfaßt, das einer geeigneten Probe entspricht.

18. Verfahren nach Anspruch 12, **dadurch gekennzeichnet, daß** die Feststellung (560) der Eignung der Probe die Bestimmung einer maximalen Geschwindigkeit der Verringerung der Konzentration des oxidativen Gases oder Dampfes in dem Bereich mit eingeschränkter Diffusion (400) umfaßt, die einer geeigneten Probe entspricht.

19. Verfahren nach Anspruch 12, **dadurch gekennzeichnet, daß** die Feststellung (560) der Eignung der Probe die Bestimmung einer minimalen Geschwindigkeit der Erhöhung der Konzentration des oxidativen Gases oder Dampfes in dem Bereich mit eingeschränkter Diffusion (400) umfaßt, die einer geeigneten Probe entspricht.

20. Verfahren nach Anspruch 12, **dadurch gekennzeichnet, daß** die Feststellung (560) der Eignung der Probe die Bestimmung einer minimalen zeitintegrierten Konzentration des oxidativen Gases oder Dampfes in dem Bereich mit eingeschränkter Diffusion (400) umfaßt, die einer geeigneten Probe entspricht.

21. Vorrichtung zur Überwachung der Konzentration eines oxidativen Gases oder Dampfes in einer Sterilisationskammer (220) während eines Sterilisationsprozesses, wobei die Vorrichtung folgendes umfaßt:
- Bereich mit eingeschränkter Diffusion (400) in Fluidkommunikation mit der Sterilisationskammer (220); und
- Konzentrationsüberwachungsgerät (10), das auf das oxidative Gas oder Dampf durch die Erzeugung eines Parameters reagiert, und mit mindestens einem Teil des Konzentrationsüberwachungsgerätes in dem Bereich mit eingeschränkter Diffusion (400), wobei das Konzentrationsüberwachungsgerät ein erstes Temperaturmeßgerät, ein zweites Temperaturmeßgerät und eine chemische Substanz (14) umfaßt, die mit dem oxidativen Gas oder Dampf reagiert, um Wärme zu erzeugen, wobei das erste Temperaturmeßgerät mit der chemischen Substanz gekoppelt ist und auf die Wärme reagiert, die von der chemischen Substanz und dem oxidativen Gas oder Dampf durch die Erzeugung eines ersten Signals erzeugt wird, und wobei das zweite Temperaturmeßgerät dafür ausgelegt ist, ein zweites Signal zu erzeugen, und wobei der Parameter in Reaktion auf das erste Signal und als weitere Reaktion auf das zweite Signal erzeugt wird,
- wobei das erste Temperaturmeßgerät eine erste Thermoelementverbindung (110) aufweist und das erste Signal eine erste Spannung umfasst, und
- wobei das zweite Temperaturmeßgerät eine zweite Thermoelementverbindung (120) aufweist und das zweite Signal eine zweite Spannung umfaßt,
**dadurch gekennzeichnet, daß** die zweite Thermoelementverbindung (120) an die erste Thermoelementverbindung (110) in Reihe gekoppelt ist und der Parameter als weitere Reaktion auf eine Nettospannung erzeugt wird, die über der ersten und zweiten Thermoelementverbindung in Reaktion auf die erste und zweite Spannung erzeugt wird.

## Revendications

1. Procédé de surveillance d'une concentration d'une vapeur ou d'un gaz oxydant dans une région à diffusion limitée (400) en communication fluide avec une chambre de stérilisation (220) lors d'un processus de stérilisation, le procédé comprenant les étapes consistant à :
prévoir un analyseur de concentration (10) qui réagit à la vapeur ou au gaz oxydant en générant un paramètre, l'analyseur de concentration comprenant un premier instrument de détection de la température, un second instrument de détection de la température et une substance chimique (14) réactive à la vapeur ou au gaz oxydant afin de produire de la chaleur, le premier instrument de détection de la température couplé à la substance chimique et qui réagit à la chaleur produite par la substance chimique et la vapeur ou le gaz oxydant en générant un premier signal, le second instrument de détection de la température générant un second signal, le paramètre généré en réponse au premier signal et en réponse supplémentaire au second signal ;
placer (520) au moins une partie de l'analyseur de concentration comprenant la substance chimique (14) dans la région à diffusion limitée (400) ;
introduire (540) la vapeur ou le gaz oxydant dans la chambre de stérilisation (220) ; et
surveiller (550) le paramètre généré par l'analyseur de concentration lors du processus de stérilisation, surveillant ainsi la concentration de la vapeur ou du gaz oxydant dans la région à diffusion limitée (400) lors du processus de stérilisation,
dans lequel le premier instrument de détection de la température comprend une première jonction de thermocouple (110) et le premier signal comprend un premier voltage, et
dans lequel le second instrument de détection de la température comprend une seconde jonction de thermocouple (120) qui génère un second voltage,
**caractérisé en ce que** la seconde jonction de thermocouple (120) est couplée en série à la première jonction de thermocouple (110), et le paramètre est généré en une réponse supplémentaire à un voltage net généré à travers les première et seconde jonctions de thermocouple en réponse aux premier et second voltages.

2. Procédé selon la revendication 1, dans lequel la surveillance du paramètre comprend en outre la conversion du paramètre généré par l'analyseur de concentration (10) en une mesure de la concentration de la vapeur ou du gaz oxydant dans la région à diffusion limitée (400).

3. Procédé selon la revendication 1, dans lequel la vapeur ou le gaz oxydant comprend du peroxyde d'hydrogène.

4. Procédé selon la revendication 1, dans lequel la région à diffusion limitée (400) comprend une région dans un jeu d'essai (430) .

5. Procédé selon la revendication 1, dans lequel la région à diffusion limitée comprend une région (500) dans un lumen (510).

6. Procédé selon la revendication 5, dans lequel le lumen (530) est dans un conteneur en communication fluide avec la chambre de (220).

7. Procédé selon la revendication 1, dans lequel la région à diffusion limitée comprend une région (600) dans un instrument de type process challenge device (PCD) (610).

8. Procédé selon la revendication 7, dans lequel le PCD (610) est ajustable afin de changer une trajectoire de diffusion entre la région dans le PCD et la chambre de stérilisation (220) et le procédé comprend en outre l'ajustement du PCD afin de reproduire une seconde région à diffusion limitée dans la charge (260).

9. Procédé selon la revendication 1, dans lequel la région à diffusion limitée comprend une région (700) dans une seconde chambre (710) en communication fluide avec la chambre de stérilisation (220).

10. Procédé selon la revendication 1, dans lequel la région à diffusion limitée (400) est conçue de façon à reproduire la diffusion de la vapeur ou du gaz oxydant vers une seconde région à diffusion limitée de la charge (260) .

11. Procédé selon la revendication 1, dans lequel la région à diffusion limitée comprend une région (800) dans un instrument prêt à utiliser (820).

12. Procédé selon la revendication 1, comprenant en outre le placement (510) d'une charge (260) dans la chambre de stérilisation (220) et la détermination (560) d'une adéquation de la charge.

13. Procédé selon la revendication 12, dans lequel l'analyseur de concentration (10) est couplé à un mécanisme de rétroaction de contrôle qui contrôle un paramètre de processus du système de stérilisation, et le procédé comprend en outre le contrôle du paramètre de processus en réponse à la détermination de l'adéquation de la charge (260).

14. Procédé selon la revendication 12, comprenant en outre l'abandon du processus de stérilisation après avoir déterminé que la charge (260) n'est pas appropriée.

15. Procédé selon la revendication 12, comprenant en outre l'introduction de la vapeur ou du gaz oxydant additionnel dans la chambre de stérilisation (220) après avoir déterminé que la charge (260) n'est pas adaptée.

16. Procédé selon la revendication 12, comprenant en outre l'activation d'une alarme après avoir déterminé que la charge (260) n'est pas adaptée.

17. Procédé selon la revendication 12, dans lequel la détermination (560) de l'adéquation de la charge comprend la définition d'un niveau de concentration minimum de la vapeur ou du gaz oxydant dans la région à diffusion limitée (400) correspondant à une charge adaptée.

18. Procédé selon la revendication 12, dans lequel la détermination (560) de l'adéquation de la charge comprend la définition d'un taux maximum de réduction de la concentration de la vapeur ou du gaz oxydant dans la région à diffusion limitée (400) correspondant à une charge adaptée.

19. Procédé selon la revendication 12, dans lequel la détermination (560) de l'adéquation de la charge comprend la définition d'un taux minimum d'augmentation de la concentration de la vapeur ou du gaz oxydant dans la région à diffusion limitée (400) correspondant à une charge adaptée.

20. Procédé selon la revendication 12, dans lequel la détermination (560) de l'adéquation de la charge comprend la définition d'une concentration intégrée en temps minimale de la vapeur ou du gaz oxydant dans la région à diffusion limitée (400) correspondant à une charge adaptée.

21. Dispositif pour surveiller une concentration d'une vapeur ou d'un gaz oxydant dans une chambre de stérilisation (220) lors d'un processus de stérilisation, l'appareil comprenant :
une région à diffusion limitée (400) en communication fluide avec la chambre de stérilisation (220) ; et
un analyseur de concentration (10) qui réagit à la vapeur ou au gaz oxydant en générant un paramètre, au moins une partie de l'analyseur de concentration dans la région à diffusion limitée (400) ; l'analyseur de concentration comprenant un premier instrument de détection de la température, un second instrument de détection de la température et une substance chimique (14) réactive avec la vapeur ou le gaz oxydant afin de produire de la chaleur, le premier instrument de détection de la température couplé à la substance chimique et qui réagit à la chaleur produite par la substance chimique et la vapeur ou le gaz oxydant en générant un premier signal, le second instrument de détection de la température adapté afin de générer un second signal, le paramètre généré en réponse au premier signal et en réponse supplémentaire au second signal,
dans lequel le premier instrument de détection de la température comprend une première jonction de thermocouple (110) et le premier signal comprend un premier voltage, et
dans lequel le second instrument de détection de la température comprend une seconde jonction de thermocouple (120) et le second signal comprend un second voltage,
**caractérisé en ce que** la seconde jonction de thermocouple (120) est couplée en série à la première jonction de thermocouple (110), et le paramètre est généré en réponse supplémentaire à un voltage net généré à travers les première et seconde jonctions de thermocouple en réponse aux premier et second voltages.
